# EUROPEAN PATENT APPLICATION

(11) **EP 1 258 495 A1**
(43) Date of publication of application: **20.11.2002**
(21) Application number: 02253262.6
(22) Date of filing: 09.05.2002
(51) Int. Cl.: C07K 14/705, C12N 15/00

(54) **98359, A sodium channel beta 4 subunit, and uses therefor**

(30) Priority: 09.05.2001 US 289893 P
(71) Applicant: Millennium Pharmaceuticals, Inc., Cambridge, Massachusetts 02139 (US)
(72) Inventor: Curtis, Rory A J, Framingham, Massachussets 01702 (US)
(74) Representative: Lunt, Mark George Francis

(57) **Abstract**

The invention provides an isolated nucleic acid molecule encoding a novel human sodium channel protein β subunit. This nucleic acid molecule encodes a transmembrane protein that bears substantially sequence similarity to mammalian sodium channel protein β subunits. The invention also provides antisense nucleic acid molecules, expression vectors containing the nucleic acid molecules of the invention, host cells into which the expression vectors have been introduced, and non-human transgenic animals in which a nucleic acid molecule of the invention has been introduced or disrupted. The invention still further provides isolated polypeptides, fusion polypeptides, antigenic peptides and antibodies. Diagnostic, screening and therapeutic methods utilizing compositions of the invention are also provided. The nucleic acids and polypeptides of the present invention are useful as modulating agents in regulating a variety of cellular processes.

SEQUENCE LISTING

<110> Curtis, Rory A.J.

<120> 98359, A SODIUM CHANNEL BETA 4 SUBUNIT,
  AND USES THEREFOR

<130> P71960EP

<150> 60/289,893
<151> 2001-05-09

<160> 12

<170> FastSEQ for Windows Version 4.0

<210> 1
<211> 4530
<212> DNA
<213> Homo sapiens

<220>
<221> CDS
<222> (154)...(837)

<400> 1
cgtccgctct gcccgctaac tttcccgagc cccgaccggc ggcgcagagc tccggggtag 60
ctttgtggcc gaacgccgac ctcgggcgga gagcgcggct gtgcccagta tcccatcccc 120
gcgacccccg cgcgctccgg agagaacagg act atg ccc ggg gct ggg gac gga 174
                                     Met Pro Gly Ala Gly Asp Gly
                                      1               5

ggc aaa gcc ccg gcg aga tgg ctg ggc act ggg ctt ttg ggc ctc ttc 222
Gly Lys Ala Pro Ala Arg Trp Leu Gly Thr Gly Leu Leu Gly Leu Phe
        10                  15                  20

ctg ctc ccc gta acc ctg tcg ctg gag gtg tct gtg gga aag gcc acc 270
Leu Leu Pro Val Thr Leu Ser Leu Glu Val Ser Val Gly Lys Ala Thr
    25                  30                  35

gac atc tac gct gtc aat ggc acg gag atc ctg ctg ccc tgc acc ttc 318
Asp Ile Tyr Ala Val Asn Gly Thr Glu Ile Leu Leu Pro Cys Thr Phe
40                  45                  50                  55

tcc agc tgc ttt ggc ttc gag gac ctc cac ttc cgg tgg acc tac aac 366
Ser Ser Cys Phe Gly Phe Glu Asp Leu His Phe Arg Trp Thr Tyr Asn
                60                  65                  70

agc agt gac gca ttc aag att ctc ata gag ggg act gtg aag aat gag 414
Ser Ser Asp Ala Phe Lys Ile Leu Ile Glu Gly Thr Val Lys Asn Glu
            75                  80                  85

aag tct gac ccc aag gtg acg ttg aaa gac gat gac cgc atc act ctg 462
Lys Ser Asp Pro Lys Val Thr Leu Lys Asp Asp Asp Arg Ile Thr Leu
        90                  95                  100

gta ggc tct act aag gag aag atg aac aac att tcc att gtg ctg agg 510
Val Gly Ser Thr Lys Glu Lys Met Asn Asn Ile Ser Ile Val Leu Arg
    105                 110                 115

gac ctg gag ttc agc gac acg ggc aaa tac acc tgc cat gtg aag aac 558
Asp Leu Glu Phe Ser Asp Thr Gly Lys Tyr Thr Cys His Val Lys Asn
120                 125                 130                 135

ccc aag gag aat aat ctc cag cac cac gcc acc atc ttc ctc caa gtc 606
Pro Lys Glu Asn Asn Leu Gln His His Ala Thr Ile Phe Leu Gln Val
                140                 145                 150

gtt gat aga ctg gaa gaa gtg gac aac aca gtg aca ctc atc atc ctg 654
Val Asp Arg Leu Glu Glu Val Asp Asn Thr Val Thr Leu Ile Ile Leu
            155                 160                 165

gct gtc gtg ggc ggg gtc atc ggg ctc ctc atc ctc atc ctg ctg atc 702
Ala Val Val Gly Gly Val Ile Gly Leu Leu Ile Leu Ile Leu Leu Ile
        170                 175                 180

aag aaa ctc atc atc ttc atc ctg aag aag act cgg gag aag aag aag 750
Lys Lys Leu Ile Ile Phe Ile Leu Lys Lys Thr Arg Glu Lys Lys Lys
    185                 190                 195

gag tgt ctc gtg agc tcc tcg ggg aat gac aac acg gag aac ggc ttg 798
Glu Cys Leu Val Ser Ser Ser Gly Asn Asp Asn Thr Glu Asn Gly Leu
200                 205                 210                 215

cct ggc tcc aag gca gag gag aaa cca cct tca aaa gtg tgagccctgc 847
Pro Gly Ser Lys Ala Glu Glu Lys Pro Pro Ser Lys Val
                220                 225

ttcgggctga gcagctgcag ggagccccct ttctgatgat gaaactgatg cttgagcccc 907
gaccgtagaa cccacgtgcc tgagacatct gctgcttggc tcaaactgta gtctttccgg 967
gcacaagaaa ccagagtcct gcccagcctg cccatcccct tcccagtcag ggctccccag 1027
ggacaaggga tggccagggg agggggtctg tggaagattc aggagaaaga aaggagaggc 1087
tagggtggtg tggaggggct ggtcccctga cacctgggca gatggggtct ccttcagtct 1147
ccctaccctg cacaagcagg gccttgattt tcctccaggc ttctcttcac aagagactgg 1207
gaggatccgt aagggatgtc ctaagagctg caccctggag atggggtgta ggaagaagtg 1267
gcttcctttg gaggtgggag tgggctggag gcctctggag aagacctggg gtgggggctg 1327
atgggggcag gcccacagtg agagactgcc tctgcttcat aggataccag atcccccaca 1387
gtcttccaag taggaaactt cctttcccct gccccgggac cctatctgcc tatcccctcc 1447
cctgctcaga gtttttaagc cctctcaacc agggctggcc accctggtct tgagggttcc 1507
tggccaccta gcctgctcct ctgctctctg ggttactgag gggctcagga aggggcccct 1567
cgagccttcc tggagtaccc gagtgctccc tatgcctttc caagcatttc tacttggaga 1627
attgggccac agaggtagtg agccagtgtc ctgggcctct gggatgcccg ccccattgct 1687
gccaatgctg gcagcccctc ccctggcatg gcaggaccat cgccactctg ggcactcctg 1747
agcccagctc tcccctgctt ctccccctcc tacctgagag gctgcaccct ccaacctccc 1807
attggctcgc tccccccgcc caccgtgccc tccatcacgc cctgccccca gggtggttca 1867
tttcccagcc ctgggtcaag ggcctgcctt cgcctcaggg actctcttcc tttggatgag 1927
ggggtccttg ggtttcccag ctgcttcctg ctcagctggg ccaccccctc ccaccctggg 1987
gttggggagg agcagggagt gggtgcccac agttttcctt tgcttctccc agagctggtt 2047
tgcacagccc ttgtgtgtgg ggctagaatg tgccttagtc ctgaatccta gcccttaccc 2107
ccatcctctc tagacggtat gtcctgacat aacagcagag tctggtgtgg tgctggtgag 2167
ggttcgccag ccctcccctc cccagggtca tagagggggc catgaggctg gaattggcca 2227
gtgactgaat cttggagatg tcggccaggt gctcccattg gggtttctag cctgccctag 2287
ggggaggtgg tgatgttggg agtgggatct cctgagtcct tgttgggcag aattggtgag 2347

gccagggatg gcagggaaaa gtggtaacaa gcctctctgc ccatctactt ccaatccctc 2407
tctcccttac tgattttttg atgccctgtc ttctgggccc ctaggaggga tgagagagga 2467
gtagccccct ttttcagaga gtttggggtc tacctcagag ctctccctgt caaaaagcag 2527
ctgcaagcct cgcaagggtg gagtgggggg agactgagga ccagtagtac ctgcagggtg 2587
cccgtggctg tggccagtgt cccttagcca acctgctggg ctcaccagtt ccccgtctga 2647
tctgcctgtg cgcctcccat tcttctctac ccagaacctg tcatgggctg gggctcagat 2707
tttcctggct ttgggagcag acagaccaga gccaccagcc attcagaaaa cttcttatag 2767
ctaccttcat gcaaaactgt tttcttcttc cttctcaatg gtgacatttg aagaggcaga 2827
gcaccttggg gctcctcctt ctgtcttaag agaaagccaa ggcacgtaga gtagggagaa 2887
gaagggcacc atcctctctt tcctccccag ggtctactgc tgatttctag atggatcatg 2947
cagcttctct cagctcagct ctttccatct accaaatggg tgtaataata cttacctacc 3007
tcacaggact gttgtgaggc ttggcaagtt ttgtctaaaa acatcttttt ggcttggaaa 3067
gggatctggg aagccaggta ttaattgcag ggatagttcc aagtctgtcc tgtcttcatc 3127
tctgtgtccc atctctacaa cccacataca gacacacaca ctctctctct ctttctttcc 3187
atcccacccc ccttggaatt atttagtctt tgcaatatta gaaaccttga ctctgatgct 3247
taaagcttct tgtccatggc ttttgtttga tggttttcaa tagaggtgac tgagattgta 3307
gggggggcat ttttggttgc ccccatgcgt gggggcacta ctaagaatgc taaacttagt 3367
ccccacaaca aagaatcatc ctgtcccatg tcaacattat acccatggag aaacactggc 3427
atggatttgc actaggatgt atatgggcaa agctatcttc cccaagtgga acctcagtgc 3487
atgcaaatct ctgatggtgg cttccagggc ttgtgggcta gagagagcca cttacaaagt 3547
cgatcttgag agacctggcc acatgcagct gggctgagtg atgtcagcga gactaaagac 3607
aaagttctga gctcctcatc aactacaaaa tatgaaatca gcattccagg ttctgggctt 3667
ctccccatgt cgtaattgaa cagaaggcag cccgaataaa cccctgatgt tagagaggcc 3727
tggggagagc agccgatggg gctcagacta catatggcag gccgatcaga gctcttgtgg 3787
agcgagggct tgagagcatg cttgtgagat ggcaggaggt ggggtgtgct tgtgtggagt 3847
gtgcgtgtgc aggcagtgtg ggtgcatggc agcgtaactg tggagtggat gggctctgca 3907
tgtaaggggt gatgcatgat gggcagatgc tggacatttg aggagccgtc tttcttggcc 3967
tgagctatgc ctgttgaggc atctggagac tgagaaagaa tcaaaggcag agaagaccag 4027
ccgtgctcct gcattccgtc actccatgac ttcatctcag tgtcacagac agctgccatc 4087
agagggctgg cagtagggag ttccaggagc ggggacttct cgggaaaatc ctataacttg 4147
ctttacttta ctttgtccca ggttggagtc cctaccctcc cacctcccac ctgatatgca 4207
gtgcttttga ctatcttatg catggtttat tcctctggct tggatgacaa caatacccat 4267
agtcaatttt cctatgtaac tatagatcaa atgatgcaac aacaggcctt gggaggcctc 4327
aggtgtgcga gtgcctctgg gaggcgcaga tgcccacaca gccagcactg acttgtgttc 4387
gagcacagaa cggatataat cagtctggcc tctacaacaa gttttgcatt gtagaattgt 4447
atttagcttt gccttggatg aaataaaaat tatgtttaat aaaaaaaaaa aaaaaagggc 4507
ggccgctaga ctagtctaaa aaa 4530

<210> 2
<211> 228
<212> PRT
<213> Homo sapiens

<220>
<221> SIGNAL
<222> (1)...(30)

<400> 2
Met Pro Gly Ala Gly Asp Gly Gly Lys Ala Pro Ala Arg Trp Leu Gly
-30 -25 -20 -15
Thr Gly Leu Leu Gly Leu Phe Leu Leu Pro Val Thr Leu Ser Leu Glu
-10 -5 1
Val Ser Val Gly Lys Ala Thr Asp Ile Tyr Ala Val Asn Gly Thr Glu
                5                   10                  15
Ile Leu Leu Pro Cys Thr Phe Ser Ser Cys Phe Gly Phe Glu Asp Leu
            20                  25                  30
His Phe Arg Trp Thr Tyr Asn Ser Ser Asp Ala Phe Lys Ile Leu Ile

35                  40                  45                  50
Glu Gly Thr Val Lys Asn Glu Lys Ser Asp Pro Lys Val Thr Leu Lys
                55                  60                  65
Asp Asp Asp Arg Ile Thr Leu Val Gly Ser Thr Lys Glu Lys Met Asn
            70                  75                  80
Asn Ile Ser Ile Val Leu Arg Asp Leu Glu Phe Ser Asp Thr Gly Lys
        85                  90                  95
Tyr Thr Cys His Val Lys Asn Pro Lys Glu Asn Asn Leu Gln His His
    100                 105                 110
Ala Thr Ile Phe Leu Gln Val Val Asp Arg Leu Glu Glu Val Asp Asn
115                 120                 125                 130
Thr Val Thr Leu Ile Ile Leu Ala Val Val Gly Gly Val Ile Gly Leu
                135                 140                 145
Leu Ile Leu Ile Leu Leu Ile Lys Lys Leu Ile Ile Phe Ile Leu Lys
            150                 155                 160
Lys Thr Arg Glu Lys Lys Lys Glu Cys Leu Val Ser Ser Ser Gly Asn
        165                 170                 175
Asp Asn Thr Glu Asn Gly Leu Pro Gly Ser Lys Ala Glu Glu Lys Pro
    180                 185                 190
Pro Ser Lys Val
        195

<210> 3
<211> 684
<212> DNA
<213> Homo sapiens

<220>
<221> CDS
<222> (1)...(684)

<400> 3
atg ccc ggg gct ggg gac gga ggc aaa gcc ccg gag aga tgg ctg ggc    48
Met Pro Gly Ala Gly Asp Gly Gly Lys Ala Pro Glu Arg Trp Leu Gly
 1               5                  10                  15

act ggg ctt ttg ggc ctn ttc ctg ctc ccc gta acc ctg tcg ctg gag    96
Thr Gly Leu Leu Gly Leu Phe Leu Leu Pro Val Thr Leu Ser Leu Glu
            20                  25                  30

gtg tct gtg gga aag gcc acc gac atc tac gct gtc aat ggc acg gag    144
Val Ser Val Gly Lys Ala Thr Asp Ile Tyr Ala Val Asn Gly Thr Glu
        35                  40                  45

atc ctg ctg ccc tgc acc ttc tcc agc tgc ttt ggc ttc gag gac ctc    192
Ile Leu Leu Pro Cys Thr Phe Ser Ser Cys Phe Gly Phe Glu Asp Leu
    50                  55                  60

cac ttc cgg tgg acc tac aac agc agt gac gca ttc aag att ctc ata    240
His Phe Arg Trp Thr Tyr Asn Ser Ser Asp Ala Phe Lys Ile Leu Ile
65                  70                  75                  80

rag ggg act gtg aag aat gag aag tct gac ccc aag gtg acg ttg aaa    288
Xaa Gly Thr Val Lys Asn Glu Lys Ser Asp Pro Lys Val Thr Leu Lys
                85                  90                  95

gac gat gac cgc atc act ctg gta ggc tct act aag gag aag atg aac     336
Asp Asp Asp Arg Ile Thr Leu Val Gly Ser Thr Lys Glu Lys Met Asn
            100                 105                 110

aac att tcc att gtg ctg agg gac ctg gag ttc agc gac acg ggc aaa     384
Asn Ile Ser Ile Val Leu Arg Asp Leu Glu Phe Ser Asp Thr Gly Lys
        115                 120                 125

tac acc tgc cat gtg aag aac ccc aag gag aat aat ctc cag cac cac     432
Tyr Thr Cys His Val Lys Asn Pro Lys Glu Asn Asn Leu Gln His His
    130                 135                 140

gcc acc atc ttc ctc caa gtc gtt gat aga ctg gaa gaa gtg gac aac     480
Ala Thr Ile Phe Leu Gln Val Val Asp Arg Leu Glu Glu Val Asp Asn
145                 150                 155                 160

aca gtg aca ctc atc atc ctg gct gtc gtg ggc ggg gtc atc ggg ctc     528
Thr Val Thr Leu Ile Ile Leu Ala Val Val Gly Gly Val Ile Gly Leu
                165                 170                 175

ctc atc ctc atc ctg ctg atc aag aaa ctc atc atc ttc atc ctg aag     576
Leu Ile Leu Ile Leu Leu Ile Lys Lys Leu Ile Ile Phe Ile Leu Lys
            180                 185                 190

aag act cgg gag aag aag aag gag tgt ctc gtg agc tcc tcg ggg aat     624
Lys Thr Arg Glu Lys Lys Lys Glu Cys Leu Val Ser Ser Ser Gly Asn
        195                 200                 205

gac aac acg gag aac ggc ttg cct ggc tcc aag gca gag gag aaa cca     672
Asp Asn Thr Glu Asn Gly Leu Pro Gly Ser Lys Ala Glu Glu Lys Pro
    210                 215                 220

cct tca aaa gtg                                                     684
Pro Ser Lys Val
225


<210> 4
<211> 45
<212> PRT
<213> Homo sapiens

<400> 4
Gly Glu Ser Val Thr Leu Thr Cys Ser Val Ser Gly Phe Gly Pro Pro
1               5                   10                  15
Pro Val Thr Trp Leu Arg Asn Gly Lys Leu Ser Leu Thr Ile Ser Val
            20                  25                  30
Thr Pro Glu Asp Ser Gly Gly Thr Tyr Thr Cys Val Val
        35                  40                  45


<210> 5
<211> 88
<212> PRT
<213> Homo sapiens

<400> 5

Gly Thr Glu Ile Leu Leu Pro Cys Thr Phe Ser Ser Cys Phe Gly Phe
1               5                   10                  15
Glu Asp Leu His Phe Arg Trp Thr Tyr Asn Ser Ser Asp Ala Phe Lys
            20                  25                  30
Ile Leu Ile Glu Gly Thr Val Lys Asn Glu Lys Ser Asp Pro Lys Val
        35                  40                  45
Thr Leu Lys Asp Asp Asp Arg Ile Thr Leu Val Gly Ser Thr Lys Glu
    50                  55                  60
Lys Met Asn Asn Ile Ser Ile Val Leu Arg Asp Leu Glu Phe Ser Asp
65                  70                  75                  80
Thr Gly Lys Tyr Thr Cys His Val
                85

<210> 6
<211> 17
<212> PRT
<213> Homo sapiens

<220>
<221> VARIANT
<222> (7)...(7)
<223> The T at position 7 can be P or R.

<221> VARIANT
<222> (9)...(9)
<223> The S at position 9 can be T or I.

<223> The amino acid at position 10 is as few as 1, up
      to 3, amino acids, and the amino acid can be any
      amino acid.

<223> The amino acid at position 12 is as few as 0, up
      to 1, amino acids, and the amino acid can be any
      amino acid.

<221> VARIANT
<222> (13)...(13)
<223> The C at position 13 can be G.

<223> The amino acid at position 14 is as few as 7, up
      to 13, amino acids, and the amino acid can be any
      amino acid.

<221> VARIANT
<222> (16)...(16)
<223> The T at position 16 can be F.

<400> 6
Gly Xaa Xaa Xaa Xaa Leu Thr Cys Ser Xaa Ser Xaa Cys Xaa Trp Thr
1               5                   10                  15
Tyr

<210> 7
<211> 10

<212> PRT
<213> Homo sapiens

<220>
<221> VARIANT
<222> (1)...(1)
<223> The S at position 1 can be D.

<221> VARIANT
<222> (2)...(2)
<223> The I at position 2 can be V or L.

<223> The amino acid at position 3 is as few as 9, up to
      10, amino acids, and the amino acid can be any
      amino acid.

<223> The amino acid at position 5 can be any amino
      acid.

<221> VARIANT
<222> (7)...(7)
<223> The T at position 7 can be N.

<223> The amino acid at position 9 can be any amino
      acid.

<221> VARIANT
<222> (10)...(10)
<223> The V at position 10 can be I.

<400> 7
Ser Ile Xaa Gly Xaa Tyr Thr Cys Xaa Val
 1               5                   10


<210> 8
<211> 218
<212> PRT
<213> Homo sapiens

<400> 8

<210> 9
<211> 215
<212> PRT
<213> Homo sapiens

<400> 9
Met His Arg Asp Ala Trp Leu Pro Arg Pro Ala Phe Ser Leu Thr Gly
 1               5                  10                  15
Leu Ser Leu Phe Phe Ser Leu Val Pro Pro Gly Arg Ser Met Glu Val
            20                  25                  30
Thr Val Pro Ala Thr Leu Asn Val Leu Asn Gly Ser Asp Ala Arg Leu
        35                  40                  45
Pro Cys Thr Phe Asn Ser Cys Tyr Thr Val Asn His Lys Gln Phe Ser
    50                  55                  60
Leu Asn Trp Thr Tyr Gln Glu Cys Asn Asn Cys Ser Glu Glu Met Phe
65                  70                  75                  80
Leu Gln Phe Arg Met Lys Ile Ile Asn Leu Lys Leu Glu Arg Phe Gln
                85                  90                  95
Asp Arg Val Glu Phe Ser Gly Asn Pro Ser Lys Tyr Asp Val Ser Val
            100                 105                 110
Met Leu Arg Asn Val Gln Pro Glu Asp Glu Gly Ile Tyr Asn Cys Tyr
        115                 120                 125
Ile Met Asn Pro Pro Asp Arg His Arg Gly His Gly Lys Ile His Leu
    130                 135                 140
Gln Val Leu Met Glu Glu Pro Pro Glu Arg Asp Ser Thr Val Ala Val
145                 150                 155                 160
Ile Val Gly Ala Ser Val Gly Gly Phe Leu Ala Val Val Ile Leu Val
                165                 170                 175
Leu Met Val Val Lys Cys Val Arg Arg Lys Lys Glu Gln Lys Leu Ser
            180                 185                 190
Thr Asp Asp Leu Lys Thr Glu Glu Glu Gly Lys Thr Asp Gly Glu Gly
        195                 200                 205
Asn Pro Asp Asp Gly Ala Lys
    210                 215

<210> 10
<211> 215
<212> PRT
<213> Rattus norvegicus

<400> 10
Met His Arg Asp Ala Trp Leu Pro Arg Pro Ala Phe Ser Leu Thr Gly

1                   5                   10                  15
Leu Ser Leu Phe Phe Ser Leu Val Pro Ser Gly Arg Ser Met Glu Val
                20                  25                  30
Thr Val Pro Thr Thr Leu Ser Val Leu Asn Gly Ser Asp Thr Arg Leu
            35                  40                  45
Pro Cys Thr Phe Asn Ser Cys Tyr Thr Val Asn His Lys Gln Phe Ser
    50                  55                  60
Leu Asn Trp Thr Tyr Gln Glu Cys Ser Asn Cys Ser Glu Glu Met Phe
65                  70                  75                  80
Leu Gln Phe Arg Met Lys Ile Ile Asn Leu Lys Leu Glu Arg Phe Gly
                85                  90                  95
Asp Arg Val Glu Phe Ser Gly Asn Pro Ser Lys Tyr Asp Val Ser Val
                100                 105                 110
Thr Leu Lys Asn Val Gln Leu Glu Asp Glu Gly Ile Tyr Asn Cys Tyr

            115                 120                 125
Ile Thr Asn Pro Pro Asp Arg His Arg Gly His Gly Lys Ile Tyr Leu
    130                 135                 140
Gln Val Leu Leu Glu Val Pro Pro Glu Arg Asp Ser Thr Val Ala Val
145                 150                 155                 160
Ile Val Gly Ala Ser Val Gly Gly Phe Leu Ala Val Val Ile Leu Val
                165                 170                 175
Leu Met Val Val Lys Cys Val Arg Arg Lys Lys Glu Gln Lys Leu Ser
                180                 185                 190
Thr Asp Asp Leu Lys Thr Glu Glu Glu Gly Lys Thr Asp Gly Glu Gly
            195                 200                 205
Asn Ala Glu Asp Gly Ala Lys
    210                 215


<210> 11
<211> 215
<212> PRT
<213> Homo sapiens

<400> 11
Met Pro Ala Phe Asn Arg Leu Phe Pro Leu Ala Ser Leu Val Leu Ile
1                   5                   10                  15
Tyr Trp Val Ser Val Cys Phe Pro Val Cys Val Glu Val Pro Ser Glu
                20                  25                  30
Thr Glu Ala Val Gln Gly Asn Pro Met Lys Leu Arg Cys Ile Ser Cys
            35                  40                  45
Met Lys Arg Glu Glu Val Glu Ala Thr Thr Val Val Glu Trp Phe Tyr
    50                  55                  60
Arg Pro Glu Gly Gly Lys Asp Phe Leu Ile Tyr Glu Tyr Arg Asn Gly
65                  70                  75                  80
His Gln Glu Val Glu Ser Pro Phe Gln Gly Arg Leu Gln Trp Asn Gly
                85                  90                  95
Ser Lys Asp Leu Gln Asp Val Ser Ile Thr Val Leu Asn Val Thr Leu
                100                 105                 110
Asn Asp Ser Gly Leu Tyr Thr Cys Asn Val Ser Arg Glu Phe Glu Phe
            115                 120                 125
Glu Ala His Arg Pro Phe Val Lys Thr Thr Arg Leu Ile Pro Leu Arg
    130                 135                 140
Val Thr Glu Glu Ala Gly Glu Asp Phe Thr Ser Val Val Ser Glu Ile
145                 150                 155                 160
Met Met Tyr Ile Leu Leu Val Phe Leu Thr Leu Trp Leu Leu Ile Glu

165                 170                 175
Met Ile Tyr Cys Tyr Arg Lys Val Ser Lys Ala Glu Glu Ala Ala Gln
            180                 185                 190
Glu Asn Ala Ser Asp Tyr Leu Ala Ile Pro Ser Glu Asn Lys Glu Asn
        195                 200                 205
Ser Ala Val Pro Val Glu Glu
    210                 215

<210> 12
<211> 215
<212> PRT
<213> Rattus norvegicus

<400> 12
Met Pro Ala Phe Asn Arg Leu Leu Pro Leu Ala Ser Leu Val Leu Ile
1               5                   10                  15
Tyr Trp Val Arg Val Cys Phe Pro Val Cys Val Glu Val Pro Ser Glu
            20                  25                  30
Thr Glu Ala Val Gln Gly Asn Pro Met Lys Leu Arg Cys Ile Ser Cys
        35                  40                  45
Met Lys Arg Glu Glu Val Glu Ala Thr Thr Val Val Glu Trp Phe Tyr
    50                  55                  60
Arg Pro Glu Gly Gly Lys Asp Phe Leu Ile Tyr Glu Tyr Arg Asn Gly
65                  70                  75                  80
His Gln Glu Val Glu Ser Pro Phe Gln Gly Arg Leu Gln Trp Asn Gly
                85                  90                  95
Ser Lys Asp Leu Gln Asp Val Ser Ile Thr Val Leu Asn Val Thr Leu
            100                 105                 110
Asn Asp Ser Gly Leu Tyr Thr Cys Asn Val Ser Arg Glu Phe Glu Phe
        115                 120                 125
Glu Ala His Arg Pro Phe Val Lys Thr Thr Arg Leu Ile Pro Leu Arg
    130                 135                 140
Val Thr Glu Glu Ala Gly Glu Asp Phe Thr Ser Val Val Ser Glu Ile
145                 150                 155                 160
Met Met Tyr Ile Leu Leu Val Phe Leu Thr Leu Trp Leu Phe Ile Glu
                165                 170                 175
Met Ile Tyr Cys Tyr Arg Lys Val Ser Lys Ala Glu Glu Ala Ala Gln
            180                 185                 190
Glu Asn Ala Ser Asp Tyr Leu Ala Ile Pro Ser Glu Asn Lys Glu Asn
        195                 200                 205
Ser Val Val Pro Val Glu Glu
    210                 215

## Description

### Cross Reference to Related Applications

This application claims the benefit of U.S. Provisional Application Number 60/289,893, filed May 9, 2001 the contents of which are incorporated herein by this reference.

### Background of the Invention

Mammalian sodium channels are integral transmembrane proteins which facilitate diffusion of sodium ions across the lipid bilayer membrane in which they are embedded. Mammalian sodium channels are composed of a large (e.g., 260 kilodalton in rat brain) pore-forming subunit designated α and one or more smaller subunits designated β (beta). The permeability of sodium channel proteins with regard to sodium ions is mediated by both the α (alpha) subunit and the β (beta) subunit(s) associated therewith.

Transmembrane flux of sodium ions is important for generation and maintenance of transmembrane action potentials which are necessary for transmission of signals along the membranes of excitable cells such as muscle and nerve cells. Voltage-sensitive (a.k.a. voltage-gated) sodium channels mediate the rapid influx of sodium ions during the rising phase of the action potential, and they also mediate re-polarization of membranes of excitable cells.

The sodium channels of excitable cells are believed to exist in three interchangeable forms. In a resting state, the sodium channel protein(s) inhibits passage of sodium ions from one side of the membrane to the other. As the membrane potential becomes less negative, the sodium channel is 'activated.' In its activated state, the sodium channel permits passage of sodium ions therethrough at a much greater rate than in its resting state. Shortly after the sodium channel is activated, it becomes 'inactivated,' in which state passage of sodium ions is once again inhibited. The sodium channel remains in the inactivated state until the membrane becomes re-polarized. Thus, the sodium channel cannot be re-activated until the membrane potential returns to approximately the value it had when the channel was in the resting state.

Rat brain sodium channels are comprised of a pore-forming α subunit and additional β subunits (McClatchey et al., (1993) *Human Mol. Genet.* 2:745-749). cDNA sequences encoding rat sodium channel α subunits in brain, skeletal muscle, and cardiac muscle have been described, as have human skeletal muscle homologs. cDNAs encoding sodium channel beta-1 subunits have been isolated from rat, human, rabbit, and mouse tissues (Isom et al. (1992) *Science* 256:839-842; McClatchey et al., *supra;* Belcher et al. (1996) *Gene* 170:285-286; Grosson et al. (1996) *Mol. Brain Res.* 42:222-226). cDNAs encoding sodium channel beta-3 subunits have been isolated from at least human and rat tissues (Morgan et al. (2000) *PNAS* 97:2308-2313).

Alteration of sodium channel subunit proteins has been associated with various human disorders in which sodium channel kinetics are altered (e.g., paramyotonia congenita and hyperkalemic periodic paralysis; McClatchey et al., *supra*), and it has furthermore been suggested that sodium channel protein abnormalities may contribute to or cause other diseases associated with generation or maintenance of membrane potential or transmembrane ion gradients (e.g., epilepsy, psychiatric diseases, and dementia; McClatchey et al., *supra;* Grosson et al., *supra;* U.S. Patent no. 5,892,018).

Need remains in the art for discovery of novel subunits of mammalian sodium channels. Such discovery enables the skilled artisan to identify disease-associated mutations, to generate and identify molecules which affect sodium channel activity by interacting with such subunits, to treat mammals afflicted with diseases caused or antagonized by mutations in such subunits, to treat mammals afflicted with diseases that can be treated by modulating activity or expression of sodium channel subunits, to deliver nucleic acid vectors encoding wild type subunits to mammals which harbor altered subunits, and to identify isoforms of and subunits related by amino acid or nucleotide homology to such subunits. The present invention satisfies these needs by providing the cDNA and amino acid sequences of a novel human sodium channel beta-4 subunit.

### Summary of the Invention

The present invention is based, at least in part, on the discovery of a cDNA molecule, designated 98359, encoding a novel sodium channel protein beta- subunit designated a beta-4 subunit, based on, among other things, amino acid homology with other sodium channel protein beta- subunits (e.g., those described in Isom et al. (1992) *Science*, 256:839-842 (rat beta-1 subunit); McClatchey et al., *supra* (human beta-1 subunit); Belcher et al. (1996) *Gene* 170:285-286 (rabbit beta-1 subunit); Grosson et al. (1996) *Mol. Brain Res.* 42:222-226 (mouse beta-1 subunit); Isom et al (1995) *Cell* 83(3):433-442 (rat beta-2 subunit); Eubanks et al. (1997) *Neuroreport*. 8(12):2775-2779 (human beta-2 subunit); and Morgan et al. (2000) *PNAS* 97(5):2308-2313 (rat and human beta-3 subunits)). The nucleotide sequence of a cDNA encoding 98359 is shown in SEQ ID NO:1, and the amino acid sequence of a 98359 polypeptide is shown in SEQ ID NO:2. In addition, the nucleotide sequence of the coding region is depicted in SEQ ID NO:3.

Accordingly, in one aspect, the invention features a nucleic acid molecule which encodes a 98359 protein or polypeptide, e.g., a biologically active portion of the 98359 protein. In a preferred embodiment, the isolated nucleic acid molecule encodes a polypeptide having the amino acid sequence of SEQ ID NO:2. In other embodiments, the invention provides isolated 98359 nucleic acid molecules having the nucleotide sequence shown in SEQ ID NO:1, SEQ ID NO:3 . In still other embodiments, the invention provides nucleic acid molecules that are substantially identical (e.g., naturally occurring allelic variants) to the nucleotide sequence shown in SEQ ID NO:1, SEQ ID NO:3. In other embodiments, the invention provides a nucleic acid molecule which hybridizes under a stringent hybridization condition to a nucleic acid molecule comprising the nucleotide sequence of SEQ ID NO:1, SEQ ID NO:3 ,wherein the nucleic acid encodes a full length 98359 protein or an active fragment thereof.

In a related aspect, the invention further provides nucleic acid constructs which include a 98359 nucleic acid molecule described herein. In certain embodiments, the nucleic acid molecules of the invention are operatively linked to native or heterologous regulatory sequences. Also included are vectors and host cells containing the 98359 nucleic acid molecules of the invention e.g., vectors and host cells suitable for producing polypeptides.

In another related aspect, the invention provides nucleic acid fragments suitable as primers or hybridization probes for the detection of 98359-encoding nucleic acids.

In still another related aspect, isolated nucleic acid molecules that are antisense to a 98359 encoding nucleic acid molecule are provided.

In another aspect, the invention features 98359 polypeptides, and biologically active or antigenic fragments thereof that are useful, e.g., as reagents or targets in assays applicable to treatment and diagnosis of sodium channel beta-4 subunit-associated or other 98359-associated disorders. In another embodiment, the invention provides 98359 polypeptides having a 98359 activity. Preferred polypeptides are 98359 proteins including at least one immunoglobulin domain, and, preferably, having a 98359 activity, e.g., a 98359 activity as described herein.

In other embodiments, the invention provides 98359 polypeptides, e.g., a 98359 polypeptide having the amino acid sequence shown in SEQ ID NO:2 ; an amino acid sequence that is substantially identical to the amino acid sequence shown in SEQ ID NO:2 ; or an amino acid sequence encoded by a nucleic acid molecule having a nucleotide sequence which hybridizes under a stringent hybridization condition to a nucleic acid molecule comprising the nucleotide sequence of SEQ ID NO:1 or SEQ ID NO:3, wherein the nucleic acid encodes a full length 98359 protein or an active fragment thereof.

In a related aspect, the invention further provides nucleic acid constructs which include a 98359 nucleic acid molecule described herein.

In a related aspect, the invention provides 98359 polypeptides or fragments operatively linked to non-98359 polypeptides to form fusion proteins.

In another aspect, the invention features antibodies and antigen-binding fragments thereof, that react with, or more preferably specifically or selectively bind 98359 polypeptides.

In another aspect, the invention provides methods of screening for compounds that modulate the expression or activity of the 98359 polypeptides or nucleic acids.

In still another aspect, the invention provides a process for modulating 98359 polypeptide or nucleic acid expression or activity, e.g., using the compounds identified in the screens described herein. In certain embodiments, the methods involve treatment of conditions related to aberrant activity or expression of the 98359 polypeptides or nucleic acids, such as conditions or disorders involving aberrant or deficient sodium channel beta-4 subunit function or expression. Examples of such disorders include, but are not limited to, human disorders in which sodium channel kinetics are altered (e.g., paramyotonia congenita and hyperkalemic periodic paralysis; McClatchey et al., *supra*), and other diseases associated with generation or maintenance of membrane potential or transmembrane ion gradients (e.g., epilepsy, psychiatric diseases, and dementia). Still other disorders in which the 98359 polypeptides or nucleic acids can be used to treat include those associated with bone metabolism, immune (e.g., inflammatory) disorders, cardiovascular disorders, endothelial cell disorders, cellular proliferative and/or differentiative disorders, liver disorders, viral diseases, pain or metabolic disorders.

The invention also provides assays for determining the activity of or the presence or absence of 98359 polypeptides or nucleic acid molecules in a biological sample, including for disease diagnosis.

In a further aspect, the invention provides assays for determining the presence or absence of a genetic alteration in a 98359 polypeptide or nucleic acid molecule, including for disease diagnosis.

In another aspect, the invention features a two dimensional array having a plurality of addresses, each address of the plurality being positionally distinguishable from each other address of the plurality, and each address of the plurality having a unique capture probe, *e.g.,* a nucleic acid or peptide sequence. At least one address of the plurality has a capture probe that recognizes a 98359 molecule. In one embodiment, the capture probe is a nucleic acid, e.g., a probe complementary to a 98359 nucleic acid sequence. In another embodiment, the capture probe is a polypeptide, *e.g.,* an antibody specific for 98359 polypeptides. Also featured is a method of analyzing a sample by contacting the sample to the aforementioned array and detecting binding of the sample to the array.

Other features and advantages of the invention will be apparent from the following detailed description, and from the claims.

### Brief Description of the Drawings

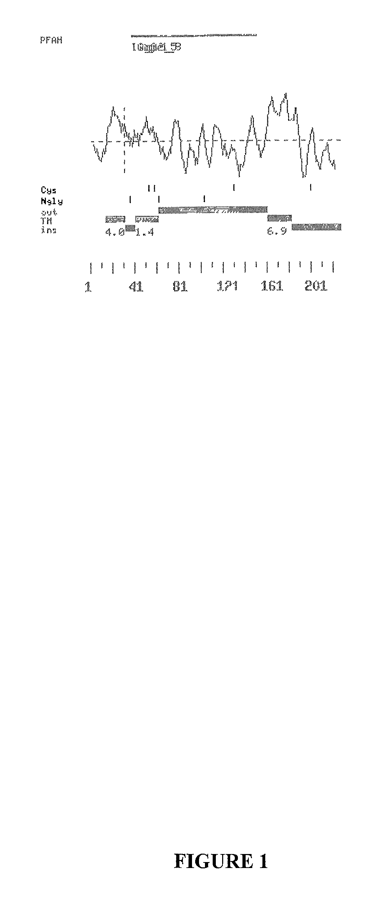

*Figure 1* depicts a hydropathy plot of human 98359. Relatively hydrophobic residues are shown above the dashed horizontal line, and relatively hydrophilic residues are below the dashed horizontal line. The cysteine residues (cys) are indicated by short vertical lines just below the hydropathy trace. The numbers corresponding to the amino acid sequence of human 98359 are indicated. Polypeptides of the invention include fragments which include: all or part of a hydrophobic sequence, e.g., a sequence above the dashed line, e.g., the sequence from about amino acid 41 to 61, from about 140 to 150, and from about 162 to 183 of SEQ ID NO:2; all or part of a hydrophilic sequence, e.g., a sequence below the dashed line, e.g., the sequence from about amino acid 62 to 140, from about 152 to 161, and from about 184 to 228 of SEQ ID NO:2; a sequence which includes a Cys, or a glycosylation site.

### Detailed Description of the Invention

The human 98359 sequence, which is approximately 4530 nucleotides long including untranslated regions, contains a predicted methionine-initiated coding sequence of about 684 nucleotides, not including the termination codon (SEQ ID NO:3). The coding sequence encodes a 228 amino acid protein (SEQ ID NO:2). The human 98359 protein of SEQ ID NO:2 and *Figure 1* includes an amino-terminal hydrophobic amino acid sequence, consistent with a signal sequence, of about 30 amino acids (from amino acid 1 to about amino acid 30 of SEQ ID NO:2, PSORT, Nakai and Kanehisa (1992) *Genomics* 14:897-911), which upon cleavage results in the production of a mature protein form). Nucleotides 154-243 encode a "pro" region which can be post-translationally cleaved. This mature protein form is approximately 198 amino acid residues in length (from about amino acid 31 to amino acid 228 of SEQ ID NO:2).

Human 98359 contains the following regions or other structural features (for general information regarding PFAM identifiers, PS prefix and PF prefix domain identification numbers, refer to Sonnhammer *et al*. (1997) *Protein* 28:405-420 and http://www.psc.edu/general/software/packages/pfam/pfam.html):

a signal peptide located at about amino acid residues 1 to 30 or SEQ ID NO:2;

an immunoglobulin domain (PFAM Accession Number PF00047) located at about amino acid residues 46 to 133 of SEQ ID NO:2;

a transmembrane domain (predicted by MEMSAT, Jones *et al.* (1994) *Biochemistry* 33:3038-3049), at about amino acids 162 to 183 of SEQ ID NO:2;

three N-glycosylation sites (Prosite PS00001) from about amino acids 45 to 48, 71 to 74, and 113 to 116 of SEQ ID NO:2;

four protein kinase C phosphorylation sites (Prosite PS00005) at about amino acids 82 to 84, 94 to 96, 106 to 108, and 126 to 128 of SEQ ID NO:2;

four casein kinase II phosphorylation sites (Prosite PS00006) located at about amino acids 94 to 97, 106 to 109, 206 to 209, and 218 to 221 of SEQ ID NO:2;

five N-myristoylation sites (Prosite PS00008) from about amino acids 3 to 8, 82 to 87, 171 to 176, 207 to 212, and 214 to 219 of SEQ ID NO:2; and

one microbodies C-terminal targeting signal site (Prosite PS00342) from about amino acids 226 to 228 of SEQ ID NO:2.

The 98359 protein contains structural characteristics in common with members of the sodium channel beta subunit (e.g., sodium channel beta-4 subunit) family, including a signal peptide, an immunoglobulin domain, and a (relatively C-terminal) transmembrane domain. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologs of non-human origin, e.g., rat or mouse proteins. Members of a family also can have common functional characteristics.

As used herein, the term "sodium channel beta-4 subunit" includes a protein or polypeptide which is capable of sodium channel beta subunit activities, such as, for example, mediating permeability of sodium channel proteins with regard to sodium ions, and generating, altering, and maintaining transmembrane sodium ion gradients. However, the term "sodium channel beta-4 subunit" also connotes beta-4 subunit-specific activities.

A 98359 polypeptide can include a signal peptide. As used herein, a "signal sequence" includes a peptide of at least about 15-30 amino acid residues in length which occurs at the N-terminus of secretory and membrane-bound proteins and which contains at least about 70% hydrophobic amino acid residues such as alanine, leucine, isoleucine, phenylalanine, proline, tyrosine, tryptophan, or valine. In a preferred embodiment, a signal sequence contains at least about 10-40 amino acid residues, preferably about 15-30 amino acid residues, more preferably about 30 amino acid residues, and has at least about 60-80%, more preferably 65-75%, and more preferably at least about 70% hydrophobic residues. A signal sequence serves to direct a protein containing such a sequence to a lipid bilayer. Thus, in one embodiment, a 98359 protein contains a signal sequence at about amino acids 1 to 30 of SEQ ID NO:2. The signal sequence is cleaved during processing of the mature protein.

In one embodiment, a 98359 protein exists in a mature form which does not include a signal sequence. In this embodiment, the 98359 protein can have a length of about 188 (e.g., 189, 190, 191, 192, 193, 194, 195, 196, 197, or 198) amino acid residues, corresponding to a protein having an amino terminus at about residue 21 (e.g., at residues 22, 23, 24, 25, 26, 27, 28, 29, 30, or 31) and having a carboxyl terminus at about residue 228 of SEQ ID NO:2.

A 98359 polypeptide can include an "immunoglobulin domain" or regions homologous with a "immunoglobulin domain." As used herein, the term "immunoglobulin domain" includes an amino acid sequence of about 50 to 200 amino acid residues in length and having a bit score for the alignment of the sequence to the immunoglobulin domain (Prosite number PF00047) of at least 20. Domains of the immunoglobulin superfamily are found in hundreds of proteins of different functions, examples of which include antibodies, the giant muscle kinase titin (also known as connectin, which plays a number of important roles in muscle contraction and elasticity), and receptor tyrosine kinases. Immunoglobulin domains can be involved in protein-protein and protein-ligand interactions.

Preferably, an immunoglobulin domain includes at least about 50 to 200 amino acids, more preferably about 75 to 150 amino acid residues, or still more preferably about 80 to 100 amino acids; has a bit score for the alignment of the sequence to the immunoglobulin domain (HMM) of at least 10 or greater, more preferably 20 or greater, still more preferably 25 or greater; and has an E-value of 1.5e-05 or less, preferably 1.5e-06 or less, still more preferably 1.5e-07 or less.

In one embodiment, an immunoglobulin domain can have the following motif (within the domain consensus sequence):

G-Xaa(4)-L-[TPR]-C-[STI]-Xaa(n1)-S-Xaa(n2)-[CG]-Xaa(n3)-W-[TF]-Y (SEQ ID NO:6).

In another embodiment, an immunoglobulin domain can have the above-described motif and/or the following motif (within the domain consensus sequence):

[SD]-[IVL]-Xaa(n4)-G-Xaa-Y-[TN]-C-Xaa-[VI] (SEQ ID NO:7).

In the above-conserved motifs, the standard IUPAC one-letter code for the amino acids is used. Each element in the pattern is separated by a dash (-); square brackets ([ ]) indicate the particular residues that are accepted at that position; Xaa indicates that any residue is accepted at that position; numbers in parentheses (()) indicate the number of residues represented by the accompanying amino acid; and an n in parenthesis indicates a variable number of possible residues. In the above-described motifs, n1 is 1-10, preferably 1-5, more preferably 1-3; n2 is 1-10, preferably 2-8, more preferably 0-1; n3 is 5-15, preferably 6-14, more preferably 7-13; and n4 is 5-15, preferably 8-13, more preferably 9-10.

The above-described motifs correspond to about amino acids 46 to 70 and 115 to 133 of SEQ ID NO:2, respectively. The immunoglobulin domain has been assigned the PFAM Accession PF00047 (http://pfam.wustl.edu/cgibin/getdesc?acc=PF00047).

Conserved motifs can be found in the 98359 protein and the amino acid sequences of other sodium channel beta subunits. For instance, a conserved motif can be seen in human beta-1 subunit (Genbank accession number Q07699; SEQ ID NO:8) from amino acids 105-123. The conserved motifs can be seen in human beta-2 subunit (Genbank accession number 060939; SEQ ID NO:9) from amino acids 43-69 and 111-129. The conserved motifs can be seen in rat beta-2 subunit (Genbank accession number P54900; SEQ ID NO:10) from amino acids 43-69 and 111-129. The conserved motifs can be seen in human beta-3 subunit (Genbank accession number CAB76825; SEQ ID NO:11), from amino acids 38-70 and 104-122. The conserved motifs can be seen in rat beta-3 subunit (Genbank accession number CAB76838; SEQ ID NO:12) from amino acids 38-64 and 104-122.

In a preferred embodiment, a 98359 polypeptide or protein has a "immunoglobulin domain" or a region which includes at least about 50 to 200 amino acids, more preferably about 75 to 150 amino acid residues, or still more preferably about 80 to 100 amino acid residues and has at least about 60%, 70% 80% 90% 95%, 99%, or 100% homology with a "immunoglobulin domain," e.g., the immunoglobulin domain of human 98359 (e.g., residues 46 to 133 of SEQ ID NO:2)(SEQ ID NO:5).

To identify the presence of a "immunoglobulin" domain in a 98359 protein sequence, and make the determination that a polypeptide or protein of interest has a particular profile, the amino acid sequence of the protein can be searched against the Pfam database of HMMs (e.g., the Pfam database, release 2.1) using the default parameters (http://www.sanger.ac.uk/Software/Pfam/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the Pfam database can be found in Sonhammer *et al*. (1997) *Proteins* 28:405-420 and a detailed description of HMMs can be found, for example, in Gribskov *et al*. (1990) *Meth. Enzymol*. 183:146-159; Gribskov *et al*. (1987) *Proc. Natl. Acad. Sci. USA* 84:4355-4358; Krogh *et al*. (1994) *J. Mol. Biol.* 235:1501-1531; and Stultz *et al*. (1993) *Protein Sci.* 2:305-314, the contents of which are incorporated herein by reference. A search was performed against the HMM database resulting in the identification of a "immunoglobulin domain" domain in the amino acid sequence of human 98359 at about residues 46-133 of SEQ ID NO:2 (SEQ ID NO:5).

A 98359 polypeptide can include at least one or more "transmembrane domain", or regions homologous with "a transmembrane domain". As used herein, the term "transmembrane domain" includes an amino acid sequence of about 10 to 40 amino acid residues in length and spans the plasma membrane. Transmembrane domains are rich in hydrophobic residues, e.g., at least 50%, 60%, 70%, 80%, 90%, 95% or more of the amino acids of a transmembrane domain are hydrophobic, e.g., leucines, isoleucines, tyrosines, or tryptophans. Transmembrane domains typically have alpha-helical structures and are described in, for example, Zagotta *et al*., (1996) *Annual Rev. Neurosci.* 19:235-263, the contents of which are incorporated herein by reference. The transmembrane domain of human 98359 is located at about residues 162-183 of SEQ ID NO:2.

In a preferred embodiment, a 98359 polypeptide or protein has at least one or more "transmembrane domains" or regions which includes at least about 12 to 35, more preferably about 14 to 30, or 15 to 25 amino acid residues and has at least about 60%, 70% 80% 90% 95%, 99%, or 100% homology with a "transmembrane domain," e.g., the transmembrane domains of human 98359 (e.g., residues 162-183 of SEQ ID NO:2). The transmembrane domain of human 98359 is visualized in the hydropathy plot (*Figure 1*) as a region of about 15 to 25 amino acids where the hydropathy trace is mostly above the horizontal line.

To identify the presence of a "transmembrane" domain in a 98359 protein sequence, and make the determination that a polypeptide or protein of interest has a particular profile, the amino acid sequence of the protein can be analyzed by a transmembrane prediction method that predicts the secondary structure and topology of integral membrane proteins based on the recognition of topological models (MEMSAT, Jones *et al*., (1994) *Biochemistry* 33:3038-3049).

Transmembrane domains exist in the same or similar locations in other sodium channel beta subunit family members, suggesting structural and functional similarity between them. The same program which identified 98359 transmembrane domains was used to identify the same in other sodium channel beta subunits. For instance, a transmembrane domain can be found in human beta- 1 subunit (Genbank accession number Q07699; SEQ ID NO:8) from amino acids 161-180; a transmembrane domain can be found in human beta-2 subunit (Genbank accession number 060939; SEQ ID NO:9) from amino acids 158-180; a transmembrane domain can be found in rat beta-2 subunit (Genbank accession number P54900; SEQ ID NO:10) from amino acids 158-180, a transmembrane domain can be found in human beta-3 subunit (Genbank accession number CAB76825; SEQ ID NO:11), from amino acids 160-179; and a transmembrane domain can be found in rat beta-3 subunit (Genbank accession number CAB76838; SEQ ID NO:12) from amino acids 160-179.

A 98359 polypeptide can include at least one or more, preferably two or more, "non-transmembrane regions." As used herein, the term "non-transmembrane region" includes an amino acid sequence not identified as a transmembrane domain. The non-transmembrane regions in 98359 are located at about amino acids 32-160 and 183-228 of SEQ ID NO:2.

The non-transmembrane regions of 98359 include at least one or more cytoplasmic regions. In one embodiment, a cytoplasmic region of a 98359 protein can include the C-terminus and can be a "C-terminal cytoplasmic domain," also referred to herein as a "C-terminal cytoplasmic tail." As used herein, a "C-terminal cytoplasmic domain" includes an amino acid sequence having a length of at least about 1 to 100, preferably about 1 to 75, more preferably about 1 to 50 amino acid residues and is located inside of a cell or within the cytoplasm of a cell. The N-terminal amino acid residue of a "C-terminal cytoplasmic domain" is adjacent to a C-terminal amino acid residue of a transmembrane domain in a 98359 protein. For example, a C-terminal cytoplasmic domain is located at about amino acid residues 184-228 of SEQ ID NO:2.

In a preferred embodiment, a 98359 polypeptide or protein has a C-terminal cytoplasmic domain or a region which includes at least about 1 to 100, preferably about 1 to 75, and more preferably about 1 to 50 amino acid residues and has at least about 60%, 70% 80% 90% 95%, 99%, or 100% homology with a C-terminal cytoplasmic domain," e.g., the C-terminal cytoplasmic domain of human 98359 (e.g., residues 184-228 of SEQ ID NO:2).

In another embodiment, a 98359 protein of the invention can include an "extracellular domain." As used herein, an "extracellular domain" includes an amino acid sequence having a length of at least about 1 to 300, preferably about 1 to 200, more preferably about 1 to 150 amino acid residues and is located outside of a cell or. For example, an extracellular domain is located at about amino acid residues 31-161 of SEQ ID NO:2.

In a preferred embodiment, a 98359 polypeptide or protein has an extracellular domain or a region which includes at least about 1 to 300, preferably about 1 to 200, and more preferably about 1 to 150 amino acid residues and has at least about 60%, 70% 80% 90% 95%, 99%, or 100% homology with a extracellular domain, e.g., the extracellular domain of human 98359 (e.g., residues 31-161 of SEQ ID NO:2).

In one embodiment, the 98359 proteins of the invention can be soluble (e.g., can exist in a truncated form (i.e., lacking a transmembrane or cytoplasmic domain)). Soluble forms of the protein of the invention can be used to, for example, inhibit receptor activity by interfering with the interaction between endogenous sodium channel beta subunits and their respective ligand(s).

A 98359 family member can include at least one extracellular domain; at least one immunoglobulin domain; at least one or more transmembrane domain; at least one or more, preferably two or more, non-transmembrane domains; and at least one cytoplasmic domain. Furthermore, a 98359 family member can include at least one, two, and preferably three N-glycosylation sites (Prosite PS00001); one, two, three, preferably four protein kinase C phosphorylation sites (Prosite PS00005); at least one, two, three, and preferably four casein kinase II phosphorylation sites (Prosite PS00006); at least one, two, three, four, five, and preferably six N-myristoylation sites (Prosite PS00008); and at least one microbodies C-terminal targeting signal site.

As the 98359 polypeptides of the invention can modulate 98359-mediated activities, they can be useful for developing novel diagnostic and therapeutic agents for sodium channel beta subunit (e.g., sodium channel beta-4 subunit)-associated or other 98359-associated disorders, as described below.

The 98359 polypeptides of the invention can modulate sodium channel beta-subunit activities, such as mediating permeability of sodium channel proteins with regard to sodium ions, and generating, altering, and maintaining transmembrane sodium ion gradients. Additionally, the 98359 polypeptides of the invention can modulate sodium channel beta-4 subunit activities specifically.

Sodium channel proteins are involved in generation, alteration, and maintenance of transmembrane sodium ion gradients. Changes in transmembrane sodium ion gradients enable excitable cells (e.g., nerve cells, muscle cells, and neuronal cells of the central nervous system) to transmit impulses along their lengths. Sodium channel proteins are therefore implicated in a wide variety of normal and abnormal cellular processes which involve transmission of electrochemical impulses along biological membranes. Such processes include, for example, normal and abnormal transmission of afferent and efferent nerve impulses and normal and abnormal transmission of voluntary and involuntary muscle contractile impulses, and any disorders which result from neuronal (e.g., pertaining to the central nervous system) or muscular (e.g., neuromuscular) dysfunction.

Exemplary nerve and neuronal cellular processes with which sodium channel proteins such as the beta-4 subunit described herein are involved include generation and transmission of pain and other sensory or perceptive nerve impulses, generation and maintenance of epileptic seizures, and establishment and endurance of neurodegenerative and mood disorders. Sodium channel proteins also have a role in a variety of disorders of mixed neuronal and psychological etiology including, for example, sleep disorders such as insomnia, hiccup, disorders of smell and taste, vision and eye movement disorders, hearing loss, vertigo, motor weakness, ataxias, neuropathic arthropathy disorders of the neuronal motor unit, nerve root disorders, and peripheral and hereditary neuropathies.

Exemplary muscle cell processes with which sodium channel proteins such as the beta-4 subunit described herein are involved include smooth, cardiac, striated, and skeletal muscle contraction, including normal voluntary and involuntary movements, such as heartbeat, digestion, and vascular tone. Aberrant muscular processes in which the protein of the invention have a role include, for example, arterial and renovascular hypertension, shock, cardiac insufficiency, heart failure, cardiac arrhythmias, cardiomyopathy, cardiac arrest, and skeletal muscle disorders.

98359 is homologous to a number of other sodium channel beta-subunit family members, and resembles other beta-subunits in terms of sequence identity and similarity, as well as on structural and function levels. For example, 98359 is homologous to human beta-1 subunit (Genbank accession number Q07699; SEQ ID NO:8), a sodium channel subunit described in McClatchey et al., *supra*. An alignment with 98359 and human beta-2 subunit reveals 21.4% identity over their lengths. Human beta-1 subunit contains an immunoglobulin domain consensus sequence motif described herein, and, as described in McClatchey et al., *supra,* maps to human chromosome 19.

98359 is homologous to human beta-2 subunit (Genbank accession number 060939; SEQ ID NO:9), a sodium channel subunit described in Eubanks et al., *supra.* An alignment with 98359 and human beta-2 subunit reveals 28.8% identity over their lengths. Human beta-2 subunit contains the immunoglobulin domain consensus sequence motifs described herein, and, as described in Eubanks et al., *supra*, is very similar to the rat beta-2 subunit, maps to human chromosome 11q3, and is thought to play a role in neurological diseases (e.g., demyelinating diseases such as Charcot-Marie-Tooth syndrome type 4B).

98359 is homologous to rat beta-2 subunit (Genbank accession number P54900; SEQ ID NO:10), a sodium channel subunit described in Isom et al., *supra.* An alignment with 98359 and rat beta-2 subunit reveals 28.8% identity over their lengths. Rat beta-2 subunit contains the immunoglobulin domain consensus sequence motifs described herein, and, as described in Isom et al., *supra*, is a glycoprotein with sequence similarity to the neural cell adhesion molecular contactin, and may play a role in regulation of sodium channel expression and localization in neurons.

98359 is homologous to human beta-3 subunit (Genbank accession number CAB76825; SEQ ID NO:11), a sodium channel subunit described in Morgan et al., *supra.* An alignment with 98359 and human beta-3 subunit reveals 20.9% identity over their lengths. Human beta-3 subunit contains the immunoglobulin domain consensus sequence motifs described herein, and, as described in Morgan et al., *supra,* maps to chromosome 11q23.3, is very similar to beta-1 subunits, and yet performs a distinct neurophysiological function.

98359 is homologous to rat beta-3 subunit (Genbank accession number CAB76838; SEQ ID NO:12), a sodium channel subunit described in Morgan et al., *supra.* An alignment with 98359 and rat beta-3 subunit reveals 21.3% identity over their lengths. Rat beta-3 subunit contains the immunoglobulin domain consensus sequence motifs described herein, and, as described in Morgan et al., *supra*, is very similar to beta-1 subunits, yet performs a distinct neurophysiological function.

98359 also shares homology with rat and rabbit beta-1 subunits (21.4% identical to each). The rat beta-1 subunit has Genbank accession number AAA88513, and is described in Isom et al., *supra,* and the rabbit beta-1 subunit has Genbank accession number JC4788, and is described in Belcher et al., *supra.*

Based on at least the above described sequence similarities, the 98359 molecules of the present invention belong to the sodium channel beta subunit family (e.g., the sodium channel beta-4 subunit family)(as described herein). Therefore, the 98359 polypeptides of the invention exhibit, and can modulate, 98359-mediated activities (e.g., sodium channel mediated activities), and can act as, or can be used to develop, novel diagnostic targets and therapeutic agents for prognosticating, diagnosing, preventing, inhibiting, alleviating, or curing 98359-mediated or related disorders (e.g., disorders associated with sodium channel family members (e.g., the sodium channel beta subunit (e.g., sodium beta-4 subunit family members))), as described below.

As used herein, a "98359 activity", "biological activity of 98359" or "functional activity of 98359", refers to an activity exerted by a 98359 protein, polypeptide or nucleic acid molecule on e.g., a 98359-responsive cell (e.g., a muscle or nerve cell) or on a 98359 substrate (e.g., a sodium ion), as determined *in vivo* or *in vitro.* In one embodiment, a 98359 activity is a direct activity, such as, for example, association with a 98359 target molecule, activation of a sodium channel, or polarization of a cell membrane. A "target molecule" or "binding partner" is a molecule with which a 98359 protein binds or interacts in nature, e.g., a sodium ion.

A 98359 activity can also be an indirect activity, e.g., a cellular signaling activity mediated by interaction of the 98359 protein with a 98359 receptor, or an activity mediated by interaction with a sodium channel alpha subunit. Based on the above-described sequence structures and similarities to molecules of known function, the 98359 molecules of the present invention can have similar biological activities as sodium channel beta subunit (e.g., sodium channel beta-4 subunit) family members. For example, the 98359 proteins of the present invention can have one or more of the following activities: (1) the ability to form protein-protein interactions with a naturally-occurring membrane protein (e.g., another sodium channel protein subunit such as an α subunit or another beta- subunit) with which the naturally-occurring polypeptide interacts *in vivo;* (2) the ability to bind a ligand of the naturally-occurring polypeptide; (3) the ability to bind to an intracellular target of the naturally-occurring polypeptide.

Other activities include: (1) the ability to mediate transmembrane sodium transport; (2) the ability to mediate maintenance of a transmembrane sodium ion gradient; (3) the ability to confer electrical excitability on cells, e.g., neurons; (4) the ability to modulate (e.g., activate or inactivate) the opening of a channel (e.g., an ion channel (e.g., a sodium channel)), e.g., in the case of an ion pore in the central nervous system; (5) the ability to affect transmission of impulses through excitable (e.g., nerve, neuronal, and muscle) cells; (6) the ability to modulate (e.g., increase) the permeability (e.g., the sodium permeability) of cell membranes (e.g., plasma membranes); (7) the ability to modulate the frequency with which neurons fire, e.g., by accelerating activation and inactivation kinetics of ion channels (e.g., sodium channels); (8) the ability to modulate (e.g., alleviate) a symptom of a disorder associated with aberrant transmembrane sodium ion transport; (9) the ability to be inactivated by compositions which normally inactivate the naturally-occurring polypeptide; and (10) the ability to modulate a pain signaling mechanism.

Examples of such disorders, e.g., sodium channel beta subunit (e.g., sodium channel beta-4 subunit)-associated or other 98359-associated disorders, include sodium channel disorders (myotonia fluctuans and permanens; human disorders in which sodium channel kinetics are altered (e.g., hyperkalemic periodic paralyses and paramyotonia congenita)); neurophysiological disorders and complications (e.g., neuromuscular disorders and complications such as convulsions, seizures, paralyses, and arrhythmias (e.g., cardiac arrhythmia)); other diseases associated with generation or maintenance of membrane potential or transmembrane ion gradients (e.g., epilepsy, psychiatric diseases, and dementia)); and demyelinating diseases such as Charcot-Marie-Tooth syndrome type 4B.

Thus, the 98359 molecules can act as novel diagnostic targets and therapeutic agents for controlling one or more conditions or disorders involving aberrant or deficient sodium channel beta-4 subunit function or expression (examples of such disorders include, but are not limited to, sodium channel disorders (e.g., paramyotonia congenita and hyperkalemic periodic paralysis), CNS disorders, and immune disorders.

The 98359 molecules of the invention can modulate the activities of cells in tissues where they are expressed. For example, 98359 mRNA is expressed in brain, dorsal root ganglia, muscle (including gastrointestinal muscle), heart, spinal cord, mammary gland, diaphragm, and skin. Accordingly, the 98359 molecules of the invention can act as therapeutic or diagnostic agents for neurological disorders (including central nervous system (CNS) disorders), muscular disorders, gastrointestinal disorders, mammary gland disorders (e.g., mastitis), cardiovascular disorders, diaphragm disorders, and skin disorders.

Since the 98359 mRNA is expressed in brain, dorsal root ganglia and spinal cord, the 98359 molecules can be used to treat neurological disorders. Neurological disorders include CNS, cognitive and neurodegenerative disorders. Examples of neurological disorders include, but are not limited to, autonomic function disorders such as hypertension and sleep disorders, and neuropsychiatric disorders, such as depression, schizophrenia, schizoaffective disorder, Korsakoff's psychosis, alcoholism, anxiety disorders, or phobic disorders; learning or memory disorders, *e.g.,* amnesia or age-related memory loss, attention deficit disorder, dysthymic disorder, major depressive disorder, mania, obsessive-compulsive disorder, psychoactive substance use disorders, anxiety, phobias, panic disorder, as well as bipolar affective disorder, *e.g.,* severe bipolar affective (mood) disorder (BP-1), and bipolar affective neurological disorders, *e.g.,* migraine and obesity.

Further examples of neurological disorders include, for example, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and lowflow states (global cerebral ischemia and focal cerebral ischemia); infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicella-zoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, and other infectious diseases of the nervous system; and transmissible spongiform encephalopathies (prion diseases).

Still other examples of neurological disorders include demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer's disease and Pick's disease, degenerative diseases of basal ganglia and brain stem, including Parkinsonism, idiopathic Parkinson's disease (paralysis agitans) and other Lewy diffuse body diseases, progressive supranuclear palsy, corticobasal degenration, multiple system atrophy, including striatonigral degenration, Shy-Drager syndrome, and olivopontocerebellar atrophy, and Huntington's disease, senile dementia, Gilles de la Tourette's syndrome, epilepsy, and Jakob-Creutzfieldt disease; spinocerebellar degenerations, including spinocerebellar ataxias, including Friedreich ataxia, and ataxia-telanglectasia; degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease; mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy; toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma); and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease.

Still further neurological disorders include such CNS-related disorders as cerebral edema, hydrocephalus, brain herniations, iatrogenic disease (due to, e.g., infection, toxins, or drugs), inflammations (e.g., bacterial and viral meningitis, encephalitis, and cerebral toxoplasmosis), cerebrovascular diseases (e.g., hypoxia, ischemia, and infarction, intracranial hemorrhage and vascular malformations, and hypertensive encephalopathy), and tumors (e.g., neuroglial tumors, neuronal tumors, tumors of pineal cells, meningeal tumors, primary and secondary lymphomas, intracranial tumors, and medulloblastoma), and to treat injury or trauma to the brain. Other CNS related disorders are listed in the American Psychiatric Association's Diagnostic and Statistical manual of Mental Disorders (DSM), the most current version of which is incorporated herein by reference in its entirety.

Furthermore, as the dorsal root ganglia, spinal cord, and brain are implicated in such somatosensory systems as tactile sensation and nociception (pain)(i.e., since the axons of primary afferent nociceptors enter the spinal cord via the dorsal root, contacting spinal neurons that transmit the pain signal to brain sites involved in pain perception), the 98359 molecules of the invention can play an important role in the regulation of metabolism or pain disorders. Diseases of metabolic imbalance include, but are not limited to, obesity, anorexia nervosa, cachexia, lipid disorders, and diabetes.

The 98359 molecules can provide novel diagnostic targets and therapeutic agents to control pain in a variety of disorders, diseases, or conditions which are characterized by a deregulated, e.g., upregulated or downregulated, pain response. For example, the 98359 molecules provide novel diagnostic targets and therapeutic agents to control the exaggerated pain response elicited during various forms of tissue injury, e.g., inflammation, infection, and ischemia, usually referred to as hyperalgesia (described in, for example, Fields, H.L. (1987) *Pain,* New York:McGraw-Hill). Moreover, the 98359 molecules provide novel diagnostic targets and therapeutic agents to control pain associated with muscoloskeletal disorders, e.g., joint pain; tooth pain; headaches; back and/or neck pain; or pain associated with surgery. Further examples of pain disorders include, but are not limited to, chronic pain; referred pain (partial displacement of pain sensation from the site of injury that produces it); neuropathic pain; pain related to diseases or disorders, e.g., pain caused by irritable bowel syndrome; and chest pain.

Since the 98359 mRNA is expressed in muscle, the 98359 molecules can be used to treat disorders of the muscular system (e.g., of the skeletal muscle system), including but not limited to, muscular dystrophy (e.g., Duchenne Muscular Dystrophy, Becker Muscular Dystrophy, Emery-Dreifuss Muscular Dystrophy, Limb-Girdle Muscular Dystrophy, Facioscapulohumeral Muscular Dystrophy, Myotonic Dystrophy, Oculopharyngeal Muscular Dystrophy, Distal Muscular Dystrophy, and Congenital Muscular Dystrophy), motor neuron diseases (e.g., Amyotrophic Lateral Sclerosis, Infantile Progressive Spinal Muscular Atrophy, Intermediate Spinal Muscular Atrophy, Spinal Bulbar Muscular Atrophy, and Adult Spinal Muscular Atrophy), myopathies (e.g., inflammatory myopathies (e.g., Dermatomyositis and Polymyositis), Myotonia Congenita, Paramyotonia Congenita, Central Core Disease, Nemaline Myopathy, Myotubular Myopathy, and Periodic Paralysis), and metabolic diseases of muscle (e.g., Phosphorylase Deficiency, Acid Maltase Deficiency, Phosphofructokinase Deficiency, Debrancher Enzyme Deficiency, Mitochondrial Myopathy, Carnitine Deficiency, Carnitine Palmityl Transferase Deficiency, Phosphoglycerate Kinase Deficiency, Phosphoglycerate Mutase Deficiency, Lactate Dehydrogenase Deficiency, and Myoadenylate Deaminase Deficiency).

Since the 98359 mRNA is expressed in gastrointestinal muscle, the 98359 molecules can be used to treat disorders of the gastrointestinal system, including but not limited to, congenital anomalies (e.g., diaphragmatic hernias, pyloric stenosis, gastric diverticula, and gastric dilatation), gastritis (e.g., acute mucosal inflammation, chronic fundal gastritis, chronic antral gastritis, hypertrophic gastritis, granulomatous gastritis, eosinophilic gastritis), ulcerations (e.g., peptic ulcers, gastric ulcers, and duodenal ulcers), or tumors (e.g., benign polyps, malignant carcinoma, argentaffinomas, carcinoids, gastrointestinal lymphomas, carcomas, and metastatic carcinoma).

Since the 98359 mRNA is expressed in heart, the 98359 molecules can be used to treat cardiovascular disorders. As used herein, disorders involving the heart, or "cardiovascular disease" or a "cardiovascular disorder" includes a disease or disorder which affects the cardiovascular system, e.g., the heart, the blood vessels, and/or the blood. A cardiovascular disorder can be caused by an imbalance in arterial pressure, a malfunction of the heart, or an occlusion of a blood vessel, e.g., by a thrombus. A cardiovascular disorder includes, but is not limited to disorders such as arteriosclerosis, atherosclerosis, cardiac hypertrophy, ischemia reperfusion injury, restenosis, arterial inflammation, vascular wall remodeling, ventricular remodeling, rapid ventricular pacing, coronary microembolism, tachycardia, bradycardia, pressure overload, aortic bending, coronary artery ligation, vascular heart disease, valvular disease, including but not limited to, valvular degeneration caused by calcification, rheumatic heart disease, endocarditis, or complications of artificial valves; atrial fibrillation, long-QT syndrome, congestive heart failure, sinus node dysfunction, angina, heart failure, hypertension, atrial fibrillation, atrial flutter, pericardial disease, including but not limited to, pericardial effusion and pericarditis; cardiomyopathies, e.g., dilated cardiomyopathy or idiopathic cardiomyopathy, myocardial infarction, coronary artery disease, coronary artery spasm, ischemic disease, arrhythmia, sudden cardiac death, and cardiovascular developmental disorders (*e.g.*, arteriovenous malformations, arteriovenous fistulae, raynaud's syndrome, neurogenic thoracic outlet syndrome, causalgia/reflex sympathetic dystrophy, hemangioma, aneurysm, cavernous angioma, aortic valve stenosis, atrial septal defects, atrioventricular canal, coarctation of the aorta, ebsteins anomaly, hypoplastic left heart syndrome, interruption of the aortic arch, mitral valve prolapse, ductus arteriosus, patent foramen ovale, partial anomalous pulmonary venous return, pulmonary atresia with ventricular septal defect, pulmonary atresia without ventricular septal defect, persistance of the fetal circulation, pulmonary valve stenosis, single ventricle, total anomalous pulmonary venous return, transposition of the great vessels, tricuspid atresia, truncus arteriosus, ventricular septal defects). A cardiovascular disease or disorder also can include an endothelial cell disorder.

As used herein, an "endothelial cell disorder" includes a disorder characterized by aberrant, unregulated, or unwanted endothelial cell activity, *e.g*., proliferation, migration, angiogenesis, or vascularization; or aberrant expression of cell surface adhesion molecules or genes associated with angiogenesis, *e.g.,* TIE-2, FLT and FLK. Endothelial cell disorders include tumorigenesis, tumor metastasis, psoriasis, diabetic retinopathy, endometriosis, Grave's disease, ischemic disease (*e.g.,* atherosclerosis), and chronic inflammatory diseases (*e.g.,* rheumatoid arthritis).

Since the 98359 mRNA is expressed in skin (e.g., hairy skin), the 98359 molecules can be used to skin treat disorders, including but not limited to skin cancers (e.g., melanoma, basal cell carcinoma, Kaposi's sarcoma, dermatitis, acne, anthrax, pigmentation changes, eczema, impetigo, psoriasis, rashes, and necrosis).

Further examples of disorders which can be treated and/or diagnosed by the 98350 molecules of the invention include but are not limited to cellular proliferative and/or differentiative disorders. Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin.

As used herein, the term "cancer" (also used interchangeably with the terms, "hyperproliferative" and "neoplastic") refers to cells having the capacity for autonomous growth, i.e., an abnormal state or condition characterized by rapidly proliferating cell growth. Cancerous disease states may be categorized as pathologic, i.e., characterizing or constituting a disease state, e.g., malignant tumor growth, or may be categorized as non-pathologic, i.e., a deviation from normal but not associated with a disease state, e.g., cell proliferation associated with wound repair. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

The 98359 molecules of the invention can be used to monitor, treat and/or diagnose a variety of proliferative disorders. Such disorders include hematopoietic neoplastic disorders. As used herein, the term "hematopoietic neoplastic disorders" includes diseases involving hyperplastic/neoplastic cells of hematopoietic origin, e.g., arising from myeloid, lymphoid or erythroid lineages, or precursor cells thereof. Preferably, the diseases arise from poorly differentiated acute leukemias, e.g., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus (1991) *Crit Rev. in Oncol.*/*Hemotol*. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

The 98359 molecules of the invention can be used to treat and/or diagnose a variety of immune, e.g., inflammatory, (e.g., respiratory inflammatory) disorders. Examples of immune disorders or diseases include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, inflammatory bowel disease, e.g., Crohn's disease and ulcerative colitis, aphthous ulcer, iritis, conjunctivitis, keratoconjunctivitis, asthma, allergic asthma, chronic obstructive pulmonary disease, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

The 98359 protein, fragments thereof, and derivatives and other variants of the sequence in SEQ ID NO:2 thereof are collectively referred to as "polypeptides or proteins of the invention" or "98359 polypeptides or proteins". Nucleic acid molecules encoding such polypeptides or proteins are collectively referred to as "nucleic acids of the invention" or "98359 nucleic acids."

As used herein, the term "nucleic acid molecule" includes DNA molecules (e.g., a cDNA or genomic DNA) and RNA molecules (e.g., an mRNA) and analogs of the DNA or RNA generated, e.g., by the use of nucleotide analogs. The nucleic acid molecule can be single-stranded or double-stranded, but preferably is double-stranded DNA.

The term "isolated or purified nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in *Current Protocols in Molecular Biology* (1989) John Wiley & Sons, N.Y., 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

As used herein, a "naturally-occurring" nucleic acid molecule refers to an RNA or DNA molecule having a nucleotide sequence that occurs in nature (e.g., encodes a natural protein).

As used herein, the terms "gene" and "recombinant gene" refer to nucleic acid molecules which include an open reading frame encoding a 98359 protein, preferably a mammalian 98359 protein, and can further include non-coding regulatory sequences, and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 98359 protein having less than about 30%, 20%, 10% and more preferably 5% (by dry weight), of non-98359 protein (also referred to herein as a "contaminating protein"), or of chemical precursors or non-98359 chemicals. When the 98359 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 98359 (e.g., the sequence of SEQ ID NO:1 or 3) without abolishing or more preferably, without substantially altering a biological activity, whereas an "essential" amino acid residue results in such a change. For example, amino acid residues that are conserved among the polypeptides of the present invention, e.g., those present in the immunoglobulin domain, are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a 98359 protein is preferably replaced with another amino acid residue from the same side chain family. Alternatively, in another embodiment, mutations can be introduced randomly along all or part of a 98359 coding sequence, such as by saturation mutagenesis, and the resultant mutants can be screened for 98359 biological activity to identify mutants that retain activity. Following mutagenesis of SEQ ID NO:1 or SEQ ID NO:3, the encoded protein can be expressed recombinantly and the activity of the protein can be determined.

As used herein, a "biologically active portion" of a 98359 protein includes a fragment of a 98359 protein which participates in an interaction between a 98359 molecule and a non-98359 molecule. Biologically active portions of a 98359 protein include peptides comprising amino acid sequences sufficiently homologous to or derived from the amino acid sequence of the 98359 protein, e.g., the amino acid sequence shown in SEQ ID NO:2, which include fewer amino acids than the full length 98359 protein, and exhibit at least one activity of a 98359 protein. Typically, biologically active portions comprise a domain or motif with at least one activity of the 98359 protein, e.g., an ability to mediate transmembrane sodium transport, and/or to mediate maintenance of a transmembrane sodium ion gradient. A biologically active portion of a 98359 protein can be a polypeptide which is, for example, 10, 25, 50, 100, 200 or more amino acids in length. Biologically active portions of a 98359 protein can be used as targets for developing agents which modulate a 98359 mediated activity, e.g., an ability to mediate transmembrane sodium transport, and/or to mediate maintenance of a transmembrane sodium ion gradient.

Calculations of homology or sequence identity (the terms "homology" and "identity" are used interchangeably herein) between sequences are performed as follows:

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence (e.g., when aligning a second sequence to the 98359 amino acid sequence of SEQ ID NO:2 having 228 amino acid residues, at least 70, preferably at least 91, more preferably at least 114, even more preferably at least 138, and even more preferably at least 160, 183, or 206 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (1970) *J. Mol. Biol.* 48:444-453 algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a Blossum 62 scoring matrix with a gap penalty of 12, a gap extend penalty of 4, and a frameshift gap penalty of 5.

The percent identity between two amino acid or nucleotide sequences can be determined using the algorithm of Meyers and Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul *et al*. (1990) *J. Mol. Biol.* 215:403-10. BLAST nucleotide searches can be performed with the NBLAST program, score = 100, wordlength = 12 to obtain nucleotide sequences homologous to 98359 nucleic acid molecules of the invention. BLAST protein searches can be performed with the XBLAST program, score = 50, wordlength = 3 to obtain amino acid sequences homologous to 98359 protein molecules of the invention. To obtain gapped alignments for comparison purposes, Gapped BLAST can be utilized as described in Altschul *et al*., (1997) *Nucleic Acids Res.* 25:3389-3402. When utilizing BLAST and Gapped BLAST programs, the default parameters of the respective programs (e.g., XBLAST and NBLAST) can be used. See http://www.ncbi.nlm.nih.gov.

Particular 98359 polypeptides of the present invention have an amino acid sequence substantially identical to the amino acid sequence of SEQ ID NO:2. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 90%, 91%, 92%, 93%, 94%, 95%, 96%, 97%, 98% or 99% identity to SEQ ID NO:2 are termed substantially identical.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 60%, or 65% identity, likely 75% identity, more likely 85%, 90%. 91%, 92%, 93%, 94%, 95%, 96%, 97%, 98% or 99% identity to SEQ ID NO:1 or 3 are termed substantially identical.

"Misexpression or aberrant expression", as used herein, refers to a non-wild type pattern of gene expression, at the RNA or protein level. It includes: expression at non-wild type levels, i.e., over or under expression; a pattern of expression that differs from wild type in terms of the time or stage at which the gene is expressed, e.g., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject", as used herein, can refer to a mammal, e.g., a human, or to an experimental or animal or disease model. The subject can also be a non-human animal, e.g., a horse, cow, goat, or other domestic animal.

A "purified preparation of cells", as used herein, refers to, in the case of plant or animal cells, an *in vitro* preparation of cells and not an entire intact plant or animal. In the case of cultured cells or microbial cells, it consists of a preparation of at least 10% and more preferably 50% of the subject cells.

Various aspects of the invention are described in further detail below.

### Isolated Nucleic Acid Molecules

In one aspect, the invention provides, an isolated or purified, nucleic acid molecule that encodes a 98359 polypeptide described herein, e.g., a full length 98359 protein or a fragment thereof, e.g., a biologically active portion of 98359 protein. Also included is a nucleic acid fragment suitable for use as a hybridization probe, which can be used, e.g., to identify a nucleic acid molecule encoding a polypeptide of the invention, 98359 mRNA, and fragments suitable for use as primers, e.g., PCR primers for the amplification or mutation of nucleic acid molecules.

In one embodiment, an isolated nucleic acid molecule of the invention includes the nucleotide sequence shown in SEQ ID NO:1, or a portion of any of this nucleotide sequence. In one embodiment, the nucleic acid molecule includes sequences encoding the human 98359 protein (i.e., "the coding region" of SEQ ID NO:1, as shown in SEQ ID NO:3), as well as 5' untranslated sequences (nucleotides 1 to 153 of SEQ ID NO:1) and 3' untranslated sequences (nucleotides 838 to 4530 of SEQ ID NO:1). Alternatively, the nucleic acid molecule can include only the coding region of SEQ ID NO:1 (e.g., SEQ ID NO:3) and, e.g., no flanking sequences which normally accompany the subject sequence. In another embodiment, the nucleic acid molecule encodes a sequence corresponding to a fragment of the protein from about amino acid 46-133 of SEQ ID NO:2.

In another embodiment, an isolated nucleic acid molecule of the invention includes a nucleic acid molecule which is a complement of the nucleotide sequence shown in SEQ ID NO:1 or SEQ ID NO:3, or a portion of any of these nucleotide sequences. In other embodiments, the nucleic acid molecule of the invention is sufficiently complementary to the nucleotide sequence shown in SEQ ID NO:1 or SEQ ID NO:3 such that it can hybridize to the nucleotide sequence shown in SEQ ID NO:1 or 3, thereby forming a stable duplex.

In one embodiment, an isolated nucleic acid molecule of the present invention includes a nucleotide sequence which is at least about: 60%, 65%, 70%, 75%, 80%, 85%, 90%, 91%, 92%, 93%, 94%, 95%, 96%, 97%, 98%, 99%, or more homologous to the entire length of the nucleotide sequence shown in SEQ ID NO: 1 or SEQ ID NO:3, or a portion, preferably of the same length, of any of these nucleotide sequences.

### 98359 Nucleic Acid Fragments

A nucleic acid molecule of the invention can include only a portion of the nucleic acid sequence of SEQ ID NO:1 or 3. For example, such a nucleic acid molecule can include a fragment which can be used as a probe or primer or a fragment encoding a portion of a 98359 protein, e.g., an immunogenic or biologically active portion of a 98359 protein. A fragment can comprise those nucleotides of SEQ ID NO:1, which encode an immunoglobulin domain of human 98359. The nucleotide sequence determined from the cloning of the 98359 gene allows for the generation of probes and primers designed for use in identifying and/or cloning other 98359 family members, or fragments thereof, as well as 98359 homologs, or fragments thereof, from other species.

In another embodiment, a nucleic acid includes a nucleotide sequence that includes part, or all, of the coding region and extends into either (or both) the 5' or 3' noncoding region. Other embodiments include a fragment which includes a nucleotide sequence encoding an amino acid fragment described herein. Nucleic acid fragments can encode a specific domain or site described herein or fragments thereof, particularly fragments thereof which are at least 100 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 98359 nucleic acid fragment can include a sequence corresponding to an immunoglobulin domain, as described herein.

98359 probes and primers are provided. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30, 35, 40, 45, 50, 55, 60, 65, or 75 consecutive nucleotides of a sense or antisense sequence of SEQ ID NO: 1 or SEQ ID NO:3, or of a naturally occurring allelic variant or mutant of SEQ ID NO:1 or SEQ ID NO:3.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes 1-30, 46-133, and 162-183 of SEQ ID NO:2

In another embodiment a set of primers is provided, e.g., primers suitable for use in a PCR, which can be used to amplify a selected region of a 98359 sequence, e.g., a domain, region, site or other sequence described herein. The primers should be at least 5, 10, or 50 base pairs in length and less than 100, or less than 200, base pairs in length. The primers should be identical, or differ by one base from a sequence disclosed herein or from a naturally occurring variant. For example, primers suitable for amplifying all or a portion of any of the following regions are provided: a signal peptide from about amino acids 1-30 of SEQ ID NO:2; transmembrane domains from about amino acids 162-183 of SEQ ID NO:2; and an immunoglobulin domain from about amino acids 46-133 of SEQ ID NO:2;

A nucleic acid fragment can encode an epitope bearing region of a polypeptide described herein.

A nucleic acid fragment encoding a "biologically active portion of a 98359 polypeptide" can be prepared by isolating a portion of the nucleotide sequence of SEQ ID NO:1 or 3, which encodes a polypeptide having a 98359 biological activity (e.g., the biological activities of the 98359 proteins are described herein), expressing the encoded portion of the 98359 protein (e.g., by recombinant expression *in vitro*) and assessing the activity of the encoded portion of the 98359 protein. For example, a nucleic acid fragment encoding a biologically active portion of 98359 includes an immunoglobulin domain, e.g., amino acid residues about 46-133 of SEQ ID NO:2. A nucleic acid fragment encoding a biologically active portion of a 98359 polypeptide, can comprise a nucleotide sequence which is greater than 250 or more nucleotides in length.

In preferred embodiments, a nucleic acid includes a nucleotide sequence which is about 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3300, 3300, 3400, 3500, 3600, 3700, 3800, 3900, 4000, 4100, 4200, 4300, 4400, 4500 or more nucleotides in length and hybridizes under stringent hybridization conditions to a nucleic acid molecule of SEQ ID NO:1 or SEQ ID NO:3.

### 98359 Nucleic Acid Variants

The invention further encompasses nucleic acid molecules that differ from the nucleotide sequence shown in SEQ ID NO:1 or SEQ ID NO:3. Such differences can be due to degeneracy of the genetic code (and result in a nucleic acid which encodes the same 98359 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2. If alignment is needed for this comparison the sequences should be aligned for maximum homology. "Looped" out sequences from deletions or insertions, or mismatches, are considered differences.

Nucleic acids of the inventor can be chosen for having codons, which are preferred, or non-preferred, for a particular expression system. E.g., the nucleic acid can be one in which at least one codon, at preferably at least 10%, or 20% of the codons has been altered such that the sequence is optimized for expression in *E. coli,* yeast, human, insect, or CHO cells.

Nucleic acid variants can be naturally occurring, such as allelic variants (same locus), homologs (different locus), and orthologs (different organism) or can be non naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO: 1 or 3, e.g., as follows: by at least one but less than 10, 20, 30, or 40 nucleotides; at least one but less than 1%, 5%, 10% or 20% of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the nucleotide sequence shown in SEQ ID NO:2 or a fragment of this sequence. Such nucleic acid molecules can readily be identified as being able to hybridize under stringent conditions, to the nucleotide sequence shown in SEQ ID NO 2 or a fragment of the sequence. Nucleic acid molecules corresponding to orthologs, homologs, and allelic variants of the 98359 cDNAs of the invention can further be isolated by mapping to the same chromosome or locus as the 98359 gene.

Preferred variants include those that are correlated with a 98359 mediated activity, e.g., an ability to mediate transmembrane sodium transport, and/or to mediate maintenance of a transmembrane sodium ion gradient.

Allelic variants of 98359, e.g., human 98359, include both functional and non-functional proteins. Functional allelic variants are naturally occurring amino acid sequence variants of the 98359 protein within a population that maintain a 98359 mediated activity, e.g., an ability to mediate transmembrane sodium transport, and/or to mediate maintenance of a transmembrane sodium ion gradient.. Functional allelic variants will typically contain only conservative substitution of one or more amino acids of SEQ ID NO:2, or substitution, deletion or insertion of non-critical residues in non-critical regions of the protein. Non-functional allelic variants are naturally-occurring amino acid sequence variants of the 98359, e.g., human 98359, protein within a population that do not have a 98359 mediated activity, e.g., an ability to mediate transmembrane sodium transport, and/or to mediate maintenance of a transmembrane sodium ion gradient.. Non-functional allelic variants will typically contain a non-conservative substitution, a deletion, or insertion, or premature truncation of the amino acid sequence of SEQ ID NO:2, or a substitution, insertion, or deletion in critical residues or critical regions of the protein.

Moreover, nucleic acid molecules encoding other 98359 family members and, thus, which have a nucleotide sequence which differs from the 98359 sequences of SEQ ID NO:1 or SEQ ID NO:3 are intended to be within the scope of the invention.

### Antisense Nucleic Acid Molecules, Ribozymes and Modified 98359 Nucleic Acid Molecules

In another aspect, the invention features, an isolated nucleic acid molecule which is antisense to 98359. An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire 98359 coding strand, or to only a portion thereof (e.g., the coding region of human 98359 corresponding to SEQ ID NO:3). In another embodiment, the antisense nucleic acid molecule is antisense to a "noncoding region" of the coding strand of a nucleotide sequence encoding 98359 (e.g., the 5' and 3' untranslated regions).

An antisense nucleic acid can be designed such that it is complementary to the entire coding region of 98359 mRNA, but more preferably is an oligonucleotide which is antisense to only a portion of the coding or noncoding region of 98359 mRNA. For example, the antisense oligonucleotide can be complementary to the region surrounding the translation start site of 98359 mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (*i.e*., RNA transcribed from the inserted nucleic acid will be of an antisense orientation to a target nucleic acid of interest, described further in the following subsection).

The antisense nucleic acid molecules of the invention are typically administered to a subject (e.g., by direct injection at a tissue site), or generated *in situ* such that they hybridize with or bind to cellular mRNA and/or genomic DNA encoding a 98359 protein to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically or selectively bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al.* (1987) *Nucleic Acids. Res.* 15:6625-6641). The antisense nucleic acid molecule can also comprise a 2'-o-methylribonucleotide (Inoue *et al.* (1987) *Nucleic Acids Res.* 15:6131-6148) or a chimeric RNA-DNA analogue (Inoue *et al*. (1987) *FEBS Lett*. 215:327-330).

In still another embodiment, an antisense nucleic acid of the invention is a ribozyme. A ribozyme having specificity for a 98359-encoding nucleic acid can include one or more sequences complementary to the nucleotide sequence of a 98359 cDNA disclosed herein (i.e., SEQ ID NO:1 or SEQ ID NO:3), and a sequence having known catalytic sequence responsible for mRNA cleavage (see U.S. Pat. No. 5,093,246 or Haselhoff and Gerlach (1988) *Nature* 334:585-591). For example, a derivative of a *Tetrahymena* L-19 IVS RNA can be constructed in which the nucleotide sequence of the active site is complementary to the nucleotide sequence to be cleaved in a 98359-encoding mRNA. See, e.g., Cech *et al*. U.S. Patent No. 4,987,071; and Cech *et al*. U.S. Patent No. 5,116,742. Alternatively, 98359 mRNA can be used to select a catalytic RNA having a specific ribonuclease activity from a pool of RNA molecules. See, e.g., Bartel and Szostak (1993) *Science* 261:1411-1418.

98359 gene expression can be inhibited by targeting nucleotide sequences complementary to the regulatory region of the 98359 (e.g., the 98359 promoter and/or enhancers) to form triple helical structures that prevent transcription of the 98359 gene in target cells. See generally, Helene (1991) *Anticancer Drug Des*. 6:569-84; Helene (1992) *Ann. N.Y. Acad. Sci.* 660:27-36; and Maher (1992) *Bioassays* 14:807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

A 98359 nucleic acid molecule can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup *et al*. (1996) *Bioorganic & Medicinal Chemistry* 4: 5-23). As used herein, the terms "peptide nucleic acid" or "PNA" refers to a nucleic acid mimic, e.g., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup *et al*. (1996) *supra;* Perry-O'Keefe *et al*. (1996) *Proc. Natl. Acad. Sci*. 93: 14670-675.

PNAs of 98359 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 98359 nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup *et al*. (1996) *supra));* or as probes or primers for DNA sequencing or hybridization (Hyrup *et al*. (1996) *supra*; Perry-O'Keefe *supra*).

In other embodiments, the oligonucleotide can include other appended groups such as peptides (e.g., for targeting host cell receptors *in vivo*), or agents facilitating transport across the cell membrane (see, e.g., Letsinger *et al*. (1989) *Proc. Natl. Acad. Sci. USA* 86:6553-6556; Lemaitre *et al.* (1987) *Proc. Natl. Acad. Sci. USA* 84:648-652; PCT Publication No. W088/09810) or the blood-brain barrier (see, e.g., PCT Publication No. W089/10134). In addition, oligonucleotides can be modified with hybridization-triggered cleavage agents (see, e.g., Krol *et al*. (1988) *Bio-Techniques* 6:958-976) or intercalating agents. (see, e.g., Zon (1988) *Pharm. Res.* 5:539-549). To this end, the oligonucleotide can be conjugated to another molecule, (e.g., a peptide, hybridization triggered cross-linking agent, transport agent, or hybridization-triggered cleavage agent).

The invention also includes molecular beacon oligonucleotide primer and probe molecules having at least one region which is complementary to a 98359 nucleic acid of the invention, two complementary regions one having a fluorophore and one a quencher such that the molecular beacon is useful for quantitating the presence of the 98359 nucleic acid of the invention in a sample. Molecular beacon nucleic acids are described, for example, in Lizardi *et al*., U.S. Patent No. 5,854,033; Nazarenko *et al*., U.S. Patent No. 5,866,336, and Livak *et al*., U.S. Patent 5,876,930.

### Isolated 98359 Polypeptides

In another aspect, the invention features, an isolated 98359 protein, or fragment, e.g., a biologically active portion, for use as immunogens or antigens to raise or test (or more generally to bind) anti-98359 antibodies. 98359 protein can be isolated from cells or tissue sources using standard protein purification techniques. 98359 protein or fragments thereof can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, e.g., cultured cells, which result in substantially the same post-translational modifications present when the polypeptide is expressed in a native cell, or in systems which result in the alteration or omission of post-translational modifications, e.g., glycosylation or cleavage, present in a native cell.

In a preferred embodiment, a 98359 polypeptide has one or more of the following characteristics:

the ability to: (1) form protein-protein interactions with a naturally-occurring membrane protein (e.g., another sodium channel protein subunit such as an α subunit or another beta- subunit) with which the naturally-occurring polypeptide interacts *in vivo;* (2) bind a ligand of the naturally-occurring polypeptide; (3) bind to an intracellular target of the naturally-occurring polypeptide.

other activities include the ability to: (1) mediate transmembrane sodium transport; (2) mediate maintenance of a transmembrane sodium ion gradient; (3) confer electrical excitability on cells, e.g., neurons; (4) modulate (e.g., activate or inactivate) the opening of a channel (e.g., an ion channel (e.g., a sodium channel)), e.g., in the case of an ion pore in the central nervous system; (5) affect transmission of impulses through excitable (e.g., nerve, neuronal, and muscle) cells; (6) modulate (e.g., increase) the permeability (e.g., the sodium permeability) of cell membranes (e.g., plasma membranes); (7) modulate the frequency with which neurons fire, e.g., by accelerating activation and inactivation kinetics of ion channels (e.g., sodium channels); (8) modulate (e.g., alleviate) a symptom of a disorder associated with aberrant transmembrane sodium ion transport; (9) be inactivated by compositions which normally inactivate the naturally-occurring polypeptide; and (10) modulate a pain signaling mechanism.

a molecular weight, e.g., a deduced molecular weight (e.g., of 25.0 kDa (22.9 kDa as a mature protein)), preferably ignoring any contribution of post translational modifications, amino acid composition or other physical characteristic of a 98359 polypeptide, e.g., a polypeptide of SEQ ID NO:2;

an overall sequence similarity of at least 60%, preferably at least 70%, more preferably at least 80, 90, or 95%, with a polypeptide of SEQ ID NO:2;

it has an immunoglobulin domain which is preferably about 70%, 75%, 80%, 85%, 90%, 95%, 96%, 97%, 98%, 99%, or 100% identical to about amino acid residues 46-133 of SEQ ID NO:2;

it has a signal peptide which is preferably about 70%, 75%, 80%, 85%, 90%, 95%, 96%, 97%, 98%, 99%, or 100% identical to about amino acid residues 1-30 of SEQ ID NO:2;

it has a transmembrane domain which is preferably about 70%, 75%, 80%, 85%, 90%, 95%, 96%, 97%, 98%, 99%, or 100% identical to about amino acid residues 162-183 of SEQ ID NO:2;

it has at least two, preferably three, and more preferably four of the cysteines found amino acid sequence of the native protein;

an expression pattern as follows, based on TaqMan analysis of 98359 expression in at least the following human tissues and cell lines (described below in the Exemplification): high levels in brain, dorsal root ganglia, and muscle (including gastrointestinal muscle, specifically); and lower levels in heart, spinal cord, and mammary glands;

an expression pattern as follows, based on TaqMan analysis of 98359 expression in at least the following rat tissues and cell lines (described below in the Exemplification): high levels in dorsal root ganglia; moderate levels in brain, spinal cord, heart, diaphragm, and muscle (including gastrointestinal muscle, specifically); and lower levels in skin (e.g., hairy skin);

an expression pattern, based on TaqMan analysis of 98359 expression, which shows downregulation of 98359 observed in the spinal cord after axotomy and slight upregulation observed in the dorsal root ganglia after axotomy, complete Freund's adjuvant (CFA) injection, and chronic constriction injury; and

an expression pattern in both monkey and rat central and peripheral nervous systems, based on *in situ hybridization* using human 98539 probes, described specifically as follows:

the following expression pattern in rat brain (based on *in situ hybridization* using human 98539 probes): high expression levels in the striatum; moderate expression levels in a subpopulation of neurons in laminae IV- VI in the cortex; further cortical expression in which the highest expression is in the pyramidal cells in layer V; expression in substantia nigra, mesencephalus, and several nuclei in the cerebellum and brain stem, including reticular, superior olive and motor nuclei; expression in motorneurons and interneurons in the spinal cord and in a subpopulation of dorsal root ganglion neurons, including some small diameter sensory neurons;

the following expression pattern in monkey tissues (based on *in situ hybridization* using human 98539 probes): high levels of expression in the claustrum and cortical layers (IV-VI) of monkey brain; expression in motorneurons and interneurons from the dorsal horn neck to the ventral horn in the monkey spinal cord; expression in a subpopulation of sensory neurons in the monkey dorsal root ganglia.

In a preferred embodiment the 98359 protein, or fragment thereof, differs from the corresponding sequence in SEQ ID NO:2. In one embodiment it differs by at least one but by less than 15, 10 or 5 amino acid residues. In another it differs from the corresponding sequence in SEQ ID NO:2 by at least one residue but less than 20%, 15%, 10% or 5% of the residues in it differ from the corresponding sequence in SEQ ID NO:2. (If this comparison requires alignment the sequences should be aligned for maximum homology. "Looped" out sequences from deletions or insertions, or mismatches, are considered differences.) The differences are, preferably, differences or changes at a non-essential residue or a conservative substitution. In a preferred embodiment the differences are not in the immunoglobulin domain at about residues 46-133 of SEQ ID NO:2. In another embodiment one or more differences are in the immunoglobulin domain at about residues 46-133 of SEQ ID NO:2.

Other embodiments include a protein that contains one or more changes in amino acid sequence, e.g., a change in an amino acid residue which is not essential for activity. Such 98359 proteins differ in amino acid sequence from SEQ ID NO:2, yet retain biological activity.

In one embodiment, the protein includes an amino acid sequence at least about 60%, 65%, 70%, 75%, 80%, 85%, 90%, 95%, 98% or more homologous to SEQ ID NO:2.

98359 proteins or fragments are provided which varies from the sequences of SEQ ID NO:2 in regions that do not correspond to a domain specifically defined herein (e.g., from about amino acids 32-45, and 133-160 of SEQ ID NO:2). (If this comparison requires alignment the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one embodiment, a biologically active portion of a 98359 protein includes an immunoglobulin domain. Moreover, other biologically active portions, in which other regions of the protein are deleted, can be prepared by recombinant techniques and evaluated for one or more of the functional activities of a native 98359 protein.

In a preferred embodiment, the 98359 protein has an amino acid sequence shown in SEQ ID NO:2. In other embodiments, the 98359 protein is sufficiently or substantially identical to SEQ ID NO:2. In yet another embodiment, the 98359 protein is sufficiently or substantially identical to SEQ ID NO:2 and retains the functional activity of the protein of SEQ ID NO:2, as described in detail in the subsections above.

### 98359 Chimeric or Fusion Proteins

In another aspect, the invention provides 98359 chimeric or fusion proteins. As used herein, a 98359 "chimeric protein" or "fusion protein" includes a 98359 polypeptide linked to a non-98359 polypeptide. A "non-98359 polypeptide" refers to a polypeptide having an amino acid sequence corresponding to a protein which is not substantially homologous to the 98359 protein, e.g., a protein which is different from the 98359 protein and which is derived from the same or a different organism. The 98359 polypeptide of the fusion protein can correspond to all or a portion e.g., a fragment described herein of a 98359 amino acid sequence. In a preferred embodiment, a 98359 fusion protein includes at least one (or two) biologically active portion of a 98359 protein. The non-98359 polypeptide can be fused to the N-terminus or C-terminus of the 98359 polypeptide.

The fusion protein can include a moiety which has a high affinity for a ligand. For example, the fusion protein can be a GST-98359 fusion protein in which the 98359 sequences are fused to the C-terminus of the GST sequences. Such fusion proteins can facilitate the purification of recombinant 98359. Alternatively, the fusion protein can be a 98359 protein containing a heterologous signal sequence at its N-terminus. In certain host cells (e.g., mammalian host cells), expression and/or secretion of 98359 can be increased through use of a heterologous signal sequence.

Fusion proteins can include all or a part of a serum protein, e.g., a portion of an immunoglobulin (e.g., IgG, IgA, or IgE), e.g., an Fc region and/or the hinge C1 and C2 sequences of an immunoglobulin or human serum albumin.

The 98359 fusion proteins of the invention can be incorporated into pharmaceutical compositions and administered to a subject *in vivo.* The 98359 fusion proteins can be used to affect the bioavailability of a 98359 substrate. 98359 fusion proteins can be useful therapeutically for the treatment of disorders caused by, for example, (i) aberrant modification or mutation of a gene encoding a 98359 protein; (ii) mis-regulation of the 98359 gene; and (iii) aberrant post-translational modification of a 98359 protein.

Moreover, the 98359-fusion proteins of the invention can be used as immunogens to produce anti-98359 antibodies in a subject, to purify 98359 ligands and in screening assays to identify molecules which inhibit the interaction of 98359 with a 98359 substrate.

Expression vectors are commercially available that already encode a fusion moiety (e.g., a GST polypeptide). A 98359-encoding nucleic acid can be cloned into such an expression vector such that the fusion moiety is linked in-frame to the 98359 protein.

### Variants of 98359 Proteins

In another aspect, the invention also features a variant of a 98359 polypeptide, e.g., which functions as an agonist (mimetics) or as an antagonist. Variants of the 98359 proteins can be generated by mutagenesis, e.g., discrete point mutation, the insertion or deletion of sequences or the truncation of a 98359 protein. An agonist of the 98359 proteins can retain substantially the same, or a subset, of the biological activities of the naturally occurring form of a 98359 protein. An antagonist of a 98359 protein can inhibit one or more of the activities of the naturally occurring form of the 98359 protein by, for example, competitively modulating a 98359-mediated activity of a 98359 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 98359 protein.

Variants of a 98359 protein can be identified by screening combinatorial libraries of mutants, e.g., truncation mutants, of a 98359 protein for agonist or antagonist activity.

Libraries of fragments e.g., N terminal, C terminal, or internal fragments, of a 98359 protein coding sequence can be used to generate a variegated population of fragments for screening and subsequent selection of variants of a 98359 protein.

Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property are known in the art. Recursive ensemble mutagenesis (REM), a new technique which enhances the frequency of functional mutants in the libraries, can be used in combination with the screening assays to identify 98359 variants (Arkin and Yourvan (1992) *Proc. Natl. Acad. Sci. USA* 89:7811-7815; Delgrave *et al*. (1993) *Protein Engineering* 6:327-331).

Cell based assays can be exploited to analyze a variegated 98359 library. For example, a library of expression vectors can be transfected into a cell line, e.g., a cell line, which ordinarily responds to 98359 in a substrate-dependent manner. The transfected cells are then contacted with 98359 and the effect of the expression of the mutant on signaling by the 98359 substrate can be detected, e.g., by measuring the influx of sodium ions during the rising phase of the action potential, and/or by measuring the re-polarization of membranes of excitable cells. Plasmid DNA can then be recovered from the cells which score for inhibition, or alternatively, potentiation of signaling by the 98359 substrate, and the individual clones further characterized.

In another aspect, the invention features a method of making a 98359 polypeptide, e.g., a peptide having a non-wild type activity, e.g., an antagonist, agonist, or super agonist of a naturally occurring 98359 polypeptide, e.g., a naturally occurring 98359 polypeptide. The method includes altering the sequence of a 98359 polypeptide, e.g., altering the sequence, e.g., by substitution or deletion of one or more residues of a non-conserved region, a domain or residue disclosed herein, and testing the altered polypeptide for the desired activity.

In another aspect, the invention features a method of making a fragment or analog of a 98359 polypeptide a biological activity of a naturally occurring 98359 polypeptide. The method includes altering the sequence, e.g., by substitution or deletion of one or more residues, of a 98359 polypeptide, e.g., altering the sequence of a non-conserved region, or a domain or residue described herein, and testing the altered polypeptide for the desired activity.

### Anti-98359 Antibodies

In another aspect, the invention provides an anti-98359 antibody. The term "antibody" as used herein refers to an immunoglobulin molecule or immunologically active portion thereof, i.e., an antigen-binding portion. Examples of immunologically active portions of immunoglobulin molecules include scFV and dcFV fragments, Fab and F(ab')₂ fragments which can be generated by treating the antibody with an enzyme such as papain or pepsin, respectively.

The antibody can be a polyclonal, monoclonal, recombinant, e.g., a chimeric or humanized, fully human, non-human, e.g., murine, or single chain antibody. In a preferred embodiment it has effector function and can fix complement. The antibody can be coupled to a toxin or imaging agent.

A full-length 98359 protein or, antigenic peptide fragment of 98359 can be used as an immunogen or can be used to identify anti-98359 antibodies made with other immunogens, e.g., cells, membrane preparations, and the like. The antigenic peptide of 98359 should include at least 8 amino acid residues of the amino acid sequence shown in SEQ ID NO:2 and encompasses an epitope of 98359. Preferably, the antigenic peptide includes at least 10 amino acid residues, more preferably at least 15 amino acid residues, even more preferably at least 20 amino acid residues, and most preferably at least 30 amino acid residues.

Fragments of 98359 which include residues about 32-40, about 183-228, and about 62-160 of SEQ ID NO:2 can be used to make, e.g., used as immunogens or used to characterize the specificity of an antibody, antibodies against hydrophilic regions of the 98359 protein (see *Figure 1*). Similarly, fragments of 98359 which include residues about 14-31 and 161-182 of SEQ ID NO:2 can be used to make an antibody against a hydrophobic region of the 98359 protein; fragments of 98359 which include residues 62-160, or a subset thereof, e.g., about residues 80-100, or about residues 140-160 of SEQ ID NO:2, can be used to make an antibody against an extracellular region of the 98359 protein; fragments of 98359 which include residues about 32-40 or about 183-228 (e.g., about residues 190-210, or about residues 205-225) of SEQ ID NO:2 can be used to make an antibody against an intracellular region of the 98359 protein; a fragment of 98359 which include residues about 50-70, about 80-100, or about 110-130 of SEQ ID NO:2 can be used to make an antibody against the immunoglobulin region of the 98359 protein.

Antibodies reactive with, or specific or selective for, any of these regions, or other regions or domains described herein are provided.

Preferred epitopes encompassed by the antigenic peptide are regions of 98359 located on the surface of the protein, e.g., hydrophilic regions, as well as regions with high antigenicity. For example, an Emini surface probability analysis of the human 98359 protein sequence can be used to indicate the regions that have a particularly high probability of being localized to the surface of the 98359 protein and are thus likely to constitute surface residues useful for targeting antibody production.

In a preferred embodiment the antibody can bind to the extracellular portion of the 98359 protein, e.g., it can bind to a whole cell which expresses the 98359 protein. In another embodiment, the antibody binds an intracellular portion of the 98359 protein.

In a preferred embodiment the antibody binds an epitope on any domain or region on 98359 proteins described herein.

Additionally, chimeric, humanized, and completely human antibodies are also within the scope of the invention. Chimeric, humanized, but most preferably, completely human antibodies are desirable for applications which include repeated administration, e.g., therapeutic treatment of human patients, and some diagnostic applications.

Chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson *et al.* International Application No. PCT/US86/02269; Akira, *et al.* European Patent Application 184,187; Taniguchi, European Patent Application 171,496; Morrison *et al.* European Patent Application 173,494; Neuberger *et al.* PCT International Publication No. WO 86/01533; Cabilly *et al.* U.S. Patent No. 4,816,567; Cabilly *et al*. European Patent Application 125,023; Better *et al*. (1988) *Science* 240:1041-1043; Liu *et al.* (1987) *Proc. Natl. Acad. Sci. USA* 84:3439-3443; Liu *et al*. (1987) *J. Immunol.* 139:3521-3526; Sun *et al*. (1987) *Proc. Natl. Acad. Sci. USA* 84:214-218; Nishimura *et al.* (1987) *Canc. Res.* 47:999-1005; Wood *et al*. (1985) *Nature* 314:446-449; and Shaw *et al*. (1988) *J. Natl. Cancer Inst*. 80:1553-1559); Morrison (1985) *Science* 229:1202-1207; Oi *et al*. (1986) *BioTechniques* 4:214; Winter U.S. Patent 5,225,539; Jones *et al*. (1986) *Nature* 321:552-525; Verhoeyan *et al*. (1988) *Science* 239:1534; and Beidler *et al*. (1988) *J. Immunol*. 141:4053-4060.

Completely human antibodies are particularly desirable for therapeutic treatment of human patients. Such antibodies can be produced using transgenic mice that are incapable of expressing endogenous immunoglobulin heavy and light chains genes, but which can express human heavy and light chain genes. *See,* for example, Lonberg and Huszar (1995) *Int. Rev. Immunol. 13*:65-93); and U.S. Patent Nos. 5,625,126; 5,633,425; 5,569,825; 5,661,016; and 5,545,806. In addition, companies such as Abgenix, Inc. (Fremont, CA) and Medarex, Inc. (Princeton, NJ), can be engaged to provide human antibodies directed against a selected antigen using technology similar to that described above.

Completely human antibodies that recognize a selected epitope can be generated using a technique referred to as "guided selection." In this approach a selected non-human monoclonal antibody, e.g., a murine antibody, is used to guide the selection of a completely human antibody recognizing the same epitope. This technology is described by Jespers *et al.* (1994) *Bio*/*Technology 12*:899-903).

The anti-98359 antibody can be a single chain antibody. A single-chain antibody (scFV) can be engineered as described in, for example, Colcher *et al*. (1999) *Ann. N Y Acad. Sci.* 880:263-80; and Reiter (1996) *Clin. Cancer Res.* 2:245-52. The single chain antibody can be dimerized or multimerized to generate multivalent antibodies having specificities for different epitopes of the same target 98359 protein.

In a preferred embodiment, the antibody has reduced or no ability to bind an Fc receptor. For example, it is an isotype or subtype, fragment or other mutant, which does not support binding to an Fc receptor, e.g., it has a mutagenized or deleted Fc receptor binding region.

An antibody (or fragment thereof) may be conjugated to a therapeutic moiety such as a cytotoxin, a therapeutic agent or a radioactive ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, e.g., maytansinol (see US Patent No. 5,208,020), CC-1065 (see US Patent Nos. 5,475,092, 5,585,499, 5,846,545) and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine, vinblastine, taxol and maytansinoids). Radioactive ions include, but are not limited to iodine, yttrium and praseodymium.

The conjugates of the invention can be used for modifying a given biological response, the therapeutic moiety is not to be construed as limited to classical chemical therapeutic agents. For example, the therapeutic moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

An anti-98359 antibody (e.g., monoclonal antibody) can be used to isolate 98359 by standard techniques, such as affinity chromatography or immunoprecipitation. Moreover, an anti-98359 antibody can be used to detect 98359 protein (e.g., in a cellular lysate or cell supernatant) in order to evaluate the abundance and pattern of expression of the protein. Anti-98359 antibodies can be used diagnostically to monitor protein levels in tissue as part of a clinical testing procedure, e.g., to determine the efficacy of a given treatment regimen. Detection can be facilitated by coupling (i.e., physically linking) the antibody to a detectable substance (i.e., antibody labelling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

In preferred embodiments, an antibody can be made by immunizing with a purified 98359 antigen, or a fragment thereof, e.g., a fragment described herein, a membrane associated antigen, tissues, e.g., crude tissue preparations, whole cells, preferably living cells, lysed cells, or cell fractions, e.g., membrane fractions.

Antibodies which bind only a native 98359 protein, only denatured or otherwise non-native 98359 protein, or which bind both, are within the invention. Antibodies with linear or conformational epitopes are within the invention. Conformational epitopes sometimes can be identified by identifying antibodies which bind to native but not denatured 98359 protein.

### Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include a 98359 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 98359 proteins, mutant forms of 98359 proteins, fusion proteins, and the like).

The recombinant expression vectors of the invention can be designed for expression of 98359 proteins in prokaryotic or eukaryotic cells. For example, polypeptides of the invention can be expressed in *E. coli*, insect cells (e.g., using baculovirus expression vectors), yeast cells or mammalian cells. Suitable host cells are discussed further in Goeddel, (1990) *Gene Expression Technology: Methods in Enzymology* 185, Academic Press, San Diego, CA . Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 98359 activity assays, (e.g., direct assays or competitive assays described in detail below), or to generate antibodies specific or selective for 98359 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (e.g., six weeks).

To maximize recombinant protein expression in *E. coli* is to express the protein in a host bacteria with an impaired capacity to proteolytically cleave the recombinant protein (Gottesman (1990) *Gene Expression Technology: Methods in Enzymology* 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada *et al*., (1992) *Nucleic Acids Res.* 20:2111-2118). Such alteration of nucleic acid sequences of the invention can be carried out by standard DNA synthesis techniques.

The 98359 expression vector can be a yeast expression vector, a vector for expression in insect cells, e.g., a baculovirus expression vector or a vector suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

In another embodiment, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert *et al*. (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J*. 8:729-733) and immunoglobulins (Banerji *et al*. (1983) *Cell* 33:729-740; Queen and Baltimore (1983) *Cell* 33:741-748), neuron-specific promoters (e.g., the neurofilament promoter; Byrne and Ruddle (1989) *Proc. Natl. Acad. Sci. USA* 86:5473-5477), pancreas-specific promoters (Edlund *et al*. (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus. For a discussion of the regulation of gene expression using antisense genes see Weintraub *et al*., (1986) *Reviews - Trends in Genetics* 1:1.

Another aspect the invention provides a host cell which includes a nucleic acid molecule described herein, e.g., a 98359 nucleic acid molecule within a recombinant expression vector or a 98359 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications can occur in succeeding generations due to either mutation or environmental influences, such progeny may not, in fact, be identical to the parent cell, but are still included within the scope of the term as used herein.

A host cell can be any prokaryotic or eukaryotic cell. For example, a 98359 protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation.

A host cell of the invention can be used to produce (i.e., express) a 98359 protein. Accordingly, the invention further provides methods for producing a 98359 protein using the host cells of the invention. In one embodiment, the method includes culturing the host cell of the invention (into which a recombinant expression vector encoding a 98359 protein has been introduced) in a suitable medium such that a 98359 protein is produced. In another embodiment, the method further includes isolating a 98359 protein from the medium or the host cell.

In another aspect, the invention features, a cell or purified preparation of cells which include a 98359 transgene, or which otherwise misexpress 98359. The cell preparation can consist of human or non-human cells, e.g., rodent cells, e.g., mouse or rat cells, rabbit cells, or pig cells. In preferred embodiments, the cell or cells include a 98359 transgene, e.g., a heterologous form of a 98359, e.g., a gene derived from humans (in the case of a non-human cell). The 98359 transgene can be misexpressed, e.g., overexpressed or underexpressed. In other preferred embodiments, the cell or cells include a gene which misexpresses an endogenous 98359, e.g., a gene the expression of which is disrupted, e.g., a knockout. Such cells can serve as a model for studying disorders which are related to mutated or misexpressed 98359 alleles or for use in drug screening.

In another aspect, the invention features, a human cell, e.g., a hematopoietic stem cell, transformed with nucleic acid which encodes a subject 98359 polypeptide.

Also provided are cells, preferably human cells, e.g., human hematopoietic or fibroblast cells, in which an endogenous 98359 is under the control of a regulatory sequence that does not normally control the expression of the endogenous 98359 gene. The expression characteristics of an endogenous gene within a cell, e.g., a cell line or microorganism, can be modified by inserting a heterologous DNA regulatory element into the genome of the cell such that the inserted regulatory element is operably linked to the endogenous 98359 gene. For example, an endogenous 98359 gene which is "transcriptionally silent," e.g., not normally expressed, or expressed only at very low levels, can be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published in May 16, 1991.

### Transgenic Animals

The invention provides non-human transgenic animals. Such animals are useful for studying the function and/or activity of a 98359 protein and for identifying and/or evaluating modulators of 98359 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA or a rearrangement, e.g., a deletion of endogenous chromosomal DNA, which preferably is integrated into or occurs in the genome of the cells of a transgenic animal. A transgene can direct the expression of an encoded gene product in one or more cell types or tissues of the transgenic animal, other transgenes, e.g., a knockout, reduce expression. Thus, a transgenic animal can be one in which an endogenous 98359 gene has been altered by, e.g., by homologous recombination between the endogenous gene and an exogenous DNA molecule introduced into a cell of the animal, e.g., an embryonic cell of the animal, prior to development of the animal.

Intronic sequences and polyadenylation signals can also be included in the transgene to increase the efficiency of expression of the transgene. A tissue-specific regulatory sequence(s) can be operably linked to a transgene of the invention to direct expression of a 98359 protein to particular cells. A transgenic founder animal can be identified based upon the presence of a 98359 transgene in its genome and/or expression of 98359 mRNA in tissues or cells of the animals. A transgenic founder animal can then be used to breed additional animals carrying the transgene. Moreover, transgenic animals carrying a transgene encoding a 98359 protein can further be bred to other transgenic animals carrying other transgenes.

98359 proteins or polypeptides can be expressed in transgenic animals or plants, e.g., a nucleic acid encoding the protein or polypeptide can be introduced into the genome of an animal. In preferred embodiments the nucleic acid is placed under the control of a tissue specific promoter, e.g., a milk or egg specific promoter, and recovered from the milk or eggs produced by the animal. Suitable animals are mice, pigs, cows, goats, and sheep.

The invention also includes a population of cells from a transgenic animal, as discussed, e.g., below.

### Uses

The nucleic acid molecules, proteins, protein homologs, and antibodies described herein can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic).

The isolated nucleic acid molecules of the invention can be used, for example, to express a 98359 protein (e.g., via a recombinant expression vector in a host cell in gene therapy applications), to detect a 98359 mRNA (e.g., in a biological sample) or a genetic alteration in a 98359 gene, and to modulate 98359 activity, as described further below. The 98359 proteins can be used to treat disorders characterized by insufficient or excessive production of a 98359 substrate or production of 98359 inhibitors. In addition, the 98359 proteins can be used to screen for naturally occurring 98359 substrates, to screen for drugs or compounds which modulate 98359 activity, as well as to treat disorders characterized by insufficient or excessive production of 98359 protein or production of 98359 protein forms which have decreased, aberrant or unwanted activity compared to 98359 wild type protein (e.g., aberrant or deficient sodium channel function or expression (e.g., various human disorders in which sodium channel kinetics are altered (e.g., paramyotonia congenita and hyperkalemic periodic paralysis)). Moreover, the anti-98359 antibodies of the invention can be used to detect and isolate 98359 proteins, regulate the bioavailability of 98359 proteins, and modulate 98359 activity.

A method of evaluating a compound for interact with, e.g., bind, a subject 98359 polypeptide is provided. The method includes: contacting the compound with the subject 98359 polypeptide; and evaluating ability of the compound to interact with, e.g., to bind or form a complex with the subject 98359 polypeptide. This method can be performed *in vitro,* e.g., in a cell free system, or *in vivo,* e.g., in a two-hybrid interaction trap assay. This method can be used to identify naturally occurring molecules which interact with subject 98359 polypeptide. It can also be used to find natural or synthetic inhibitors of subject 98359 polypeptide. Screening methods are discussed in more detail below.

### Screening Assays:

The invention provides methods (also referred to herein as "screening assays") for identifying modulators, i.e., candidate or test compounds or agents (e.g., proteins, peptides, peptidomimetics, peptoids, small molecules or other drugs) which bind to 98359 proteins, have a stimulatory or inhibitory effect on, for example, 98359 expression or 98359 activity, or have a stimulatory or inhibitory effect on, for example, the expression or activity of a 98359 substrate. Compounds thus identified can be used to modulate the activity of target gene products (e.g., 98359 genes) in a therapeutic protocol, to elaborate the biological function of the target gene product, or to identify compounds that disrupt normal target gene interactions.

In one embodiment, the invention provides assays for screening candidate or test compounds which are substrates of a 98359 protein or polypeptide or a biologically active portion thereof. In another embodiment, the invention provides assays for screening candidate or test compounds which bind to or modulate the activity of a 98359 protein or polypeptide or a biologically active portion thereof.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann *et al*. (1994) *J* .*Med. Chem.* 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) *Anticancer Drug Des.* 12:145).

Examples of methods for the synthesis of molecular libraries can be found in the art, for example in: DeWitt *et al*. (1993) *Proc. Natl. Acad. Sci. U.S.A*. 90:6909-13; Erb *et al.* (1994) *Proc. Natl. Acad. Sci. USA* 91:11422-426; Zuckermann *et al*. (1994). *J. Med. Chem.* 37:2678-85; Cho *et al*. (1993) *Science* 261:1303; Carrell *et al*. (1994) *Angew. Chem. Int. Ed. Engl*. 33:2059; Carell *et al*. (1994) *Angew*. *Chem. Int*. *Ed. Engl.* 33:2061; and in Gallop *et al.* (1994) *J. Med. Chem.* 37:1233-51.

Libraries of compounds can be presented in solution (e.g., Houghten (1992) *Biotechniques* 13:412-421), or on beads (Lam (1991) *Nature* 354:82-84), chips (Fodor (1993) *Nature* 364:555-556), bacteria (Ladner, USP 5,223,409), spores (Ladner USP '409), plasmids (Cull *et al*. (1992) *Proc Natl Acad Sci USA* 89:1865-1869) or on phage (Scott and Smith (1990) *Science* 249:386-390; Devlin (1990) *Science* 249:404-406; Cwirla *et al*. (1990) *Proc. Natl. Acad. Sci.* 87:6378-6382; Felici (1991) *J. Mol. Biol.* 222:301-310; Ladner *supra*.).

In one embodiment, an assay is a cell-based assay in which a cell which expresses a 98359 protein or biologically active portion thereof is contacted with a test compound, and the ability of the test compound to modulate 98359 activity is determined. Determining the ability of the test compound to modulate 98359 activity can be accomplished by monitoring, for example, the influx of sodium ions during the rising phase of the action potential, and/or by monitoring the re-polarization of membranes of excitable cells function. The cell, for example, can be of mammalian origin, e.g., human.

The ability of the test compound to modulate 98359 binding to a compound, e.g., a 98359 substrate, or to bind to 98359 can also be evaluated. This can be accomplished, for example, by coupling the compound, e.g., the substrate, with a radioisotope or enzymatic label such that binding of the compound, e.g., the substrate, to 98359 can be determined by detecting the labeled compound, e.g., substrate, in a complex. Alternatively, 98359 could be coupled with a radioisotope or enzymatic label to monitor the ability of a test compound to modulate 98359 binding to a 98359 substrate in a complex. For example, compounds (e.g., 98359 substrates) can be labeled with ¹²⁵I, ¹⁴C, ³⁵S or ³H., either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 98359 substrate) to interact with 98359 with or without the labeling of any of the interactants can be evaluated. For example, a microphysiometer can be used to detect the interaction of a compound with 98359 without the labeling of either the compound or the 98359. McConnell *et al*. (1992) *Science* 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 98359.

In yet another embodiment, a cell-free assay is provided in which a 98359 protein or biologically active portion thereof is contacted with a test compound and the ability of the test compound to bind to the 98359 protein or biologically active portion thereof is evaluated. Preferred biologically active portions of the 98359 proteins to be used in assays of the present invention include fragments which participate in interactions with non-98359 molecules, e.g., fragments with high surface probability scores.

Soluble and/or membrane-bound forms of isolated proteins (e.g., 98359 proteins or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, e.g., using fluorescence energy transfer (FET) (see, for example, Lakowicz *et al*., U.S. Patent No. 5,631,169; Stavrianopoulos, *et al*., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule can simply utilize the natural fluorescent energy of tryptophan residues. Labels are chosen that emit different wavelengths of light, such that the 'acceptor' molecule label can be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 98359 protein to bind to a target molecule can be accomplished using real-time Biomolecular Interaction Analysis (BIA) (see, e.g., Sjolander and Urbaniczky (1991) *Anal. Chem.* 63:2338-2345 and Szabo *et al*. (1995) *Curr. Opin. Struct. Biol*. 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 98359, an anti-98359 antibody or its target molecule to facilitate separation of complexed from uncomplexed forms of one or both of the proteins, as well as to accommodate automation of the assay. Binding of a test compound to a 98359 protein, or interaction of a 98359 protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/98359 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 98359 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 98359 binding or activity determined using standard techniques.

Other techniques for immobilizing either a 98359 protein or a target molecule on matrices include using conjugation of biotin and streptavidin. Biotinylated 98359 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific or selective for the immobilized component (the antibody, in turn, can be directly labeled or indirectly labeled with, e.g., a labeled anti-Ig antibody).

In one embodiment, this assay is performed utilizing antibodies reactive with 98359 protein or target molecules but which do not interfere with binding of the 98359 protein to its target molecule. Such antibodies can be derivatized to the wells of the plate, and unbound target or 98359 protein trapped in the wells by antibody conjugation. Methods for detecting such complexes, in addition to those described above for the GST-immobilized complexes, include immunodetection of complexes using antibodies reactive with the 98359 protein or target molecule, as well as enzyme-linked assays which rely on detecting an enzymatic activity associated with the 98359 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas and Minton (1993) *Trends Biochem Sci* 18:284-7); chromatography (gel filtration chromatography, ion-exchange chromatography); electrophoresis (see, e.g., Ausubel *et al*., eds. (1999) *Current Protocols in Molecular Biology,* J. Wiley, New York.); and immunoprecipitation (see, for example, Ausubel *et al*., eds. (1999) *Current Protocols in Molecular Biology,* J. Wiley, New York). Such resins and chromatographic techniques are known to one skilled in the art (see, e.g., Heegaard (1998) *J Mol Recognit* 11:141-8; Hage and Tweed (1997) *J Chromatogr B Biomed Sci Appl*. 699:499-525). Further, fluorescence energy transfer can also be conveniently utilized, as described herein, to detect binding without further purification of the complex from solution.

In a preferred embodiment, the assay includes contacting the 98359 protein or biologically active portion thereof with a known compound which binds 98359 to form an assay mixture, contacting the assay mixture with a test compound, and determining the ability of the test compound to interact with a 98359 protein, wherein determining the ability of the test compound to interact with a 98359 protein includes determining the ability of the test compound to preferentially bind to 98359 or biologically active portion thereof, or to modulate the activity of a target molecule, as compared to the known compound.

The target gene products of the invention can, *in vivo,* interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 98359 genes herein identified. In an alternative embodiment, the invention provides methods for determining the ability of the test compound to modulate the activity of a 98359 protein through modulation of the activity of a downstream effector of a 98359 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific or selective for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific or selective for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific or selective for one of the binding components to anchor any complexes formed in solution, and a labeled antibody specific or selective for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 98359 proteins can be used as "bait proteins" in a two-hybrid assay or three-hybrid assay (see, e.g., U.S. Patent No. 5,283,317; Zervos *et al*. (1993) *Cell* 72:223-232; Madura *et al*. (1993) *J. Biol. Chem.* 268:12046-12054; Bartel *et al*. (1993) *Biotechniques* 14:920-924; Iwabuchi *et al*. (1993) *Oncogene* 8:1693-1696; and Brent WO94/10300), to identify other proteins, which bind to or interact with 98359 ("98359-binding proteins" or "98359-bp") and are involved in 98359 activity. Such 98359-bps can be activators or inhibitors of signals by the 98359 proteins or 98359 targets as, for example, downstream elements of a 98359-mediated signaling pathway.

The two-hybrid system is based on the modular nature of most transcription factors, which consist of separable DNA-binding and activation domains. Briefly, the assay utilizes two different DNA constructs. In one construct, the gene that codes for a 98359 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 98359 protein can be the fused to the activator domain.) If the "bait" and the "prey" proteins are able to interact, *in vivo,* forming a 98359-dependent complex, the DNA-binding and activation domains of the transcription factor are brought into close proximity. This proximity allows transcription of a reporter gene (e.g., lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 98359 protein.

In another embodiment, modulators of 98359 expression are identified. For example, a cell or cell free mixture is contacted with a candidate compound and the expression of 98359 mRNA or protein evaluated relative to the level of expression of 98359 mRNA or protein in the absence of the candidate compound. When expression of 98359 mRNA or protein is greater in the presence of the candidate compound than in its absence, the candidate compound is identified as a stimulator of 98359 mRNA or protein expression. Alternatively, when expression of 98359 mRNA or protein is less (statistically significantly less) in the presence of the candidate compound than in its absence, the candidate compound is identified as an inhibitor of 98359 mRNA or protein expression. The level of 98359 mRNA or protein expression can be determined by methods described herein for detecting 98359 mRNA or protein.

In another aspect, the invention pertains to a combination of two or more of the assays described herein. For example, a modulating agent can be identified using a cell-based or a cell free assay, and the ability of the agent to modulate the activity of a 98359 protein can be confirmed *in vivo,* e.g., in an animal such as an animal model for aberrant or deficient sodium channel beta subunit function or expression.

This invention further pertains to novel agents identified by the above-described screening assays. Accordingly, it is within the scope of this invention to further use an agent identified as described herein (e.g., a 98359 modulating agent, an antisense 98359 nucleic acid molecule, a 98359-specific antibody, or a 98359-binding partner) in an appropriate animal model to determine the efficacy, toxicity, side effects, or mechanism of action, of treatment with such an agent. Furthermore, novel agents identified by the above-described screening assays can be used for treatments as described herein.

### Detection Assays

Portions or fragments of the nucleic acid sequences identified herein can be used as polynucleotide reagents. For example, these sequences can be used to: (i) map their respective genes on a chromosome e.g., to locate gene regions associated with genetic disease or to associate 98359 with a disease; (ii) identify an individual from a minute biological sample (tissue typing); and (iii) aid in forensic identification of a biological sample. These applications are described in the subsections below.

### Chromosome Mapping

The 98359 nucleotide sequences or portions thereof can be used to map the location of the 98359 genes on a chromosome. This process is called chromosome mapping. Chromosome mapping is useful in correlating the 98359 sequences with genes associated with disease.

Briefly, 98359 genes can be mapped to chromosomes by preparing PCR primers (preferably 15-25 bp in length) from the 98359 nucleotide sequences. These primers can then be used for PCR screening of somatic cell hybrids containing individual human chromosomes. Only those hybrids containing the human gene corresponding to the 98359 sequences will yield an amplified fragment.

A panel of somatic cell hybrids in which each cell line contains either a single human chromosome or a small number of human chromosomes, and a full set of mouse chromosomes, can allow easy mapping of individual genes to specific human chromosomes. (D'Eustachio *et al.* (1983) *Science* 220:919-924).

Other mapping strategies e.g., in situ hybridization (described in Fan *et al*. (1990) *Proc. Natl. Acad. Sci. USA*, 87:6223-27), pre-screening with labeled flow-sorted chromosomes, and pre-selection by hybridization to chromosome specific cDNA libraries can be used to map 98359 to a chromosomal location.

Fluorescence *in situ* hybridization (FISH) of a DNA sequence to a metaphase chromosomal spread can further be used to provide a precise chromosomal location in one step. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma *et al*. (1988) Human Chromosomes: A Manual of Basic Techniques, Pergamon Press, New York).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in McKusick, *Mendelian Inheritance in Man,* available on-line through Johns Hopkins University Welch Medical Library). The relationship between a gene and a disease, mapped to the same chromosomal region, can then be identified through linkage analysis (co-inheritance of physically adjacent genes), described in, for example, Egeland *et al*. (1987) *Nature,* 325:783-787.

Moreover, differences in the DNA sequences between individuals affected and unaffected with a disease associated with the 98359 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

98359 sequences can be used to identify individuals from biological samples using, e.g., restriction fragment length polymorphism (RFLP). In this technique, an individual's genomic DNA is digested with one or more restriction enzymes, the fragments separated, e.g., in a Southern blot, and probed to yield bands for identification. The sequences of the present invention are useful as additional DNA markers for RFLP (described in U.S. Patent 5,272,057).

Furthermore, the sequences of the present invention can also be used to determine the actual base-by-base DNA sequence of selected portions of an individual's genome. Thus, the 98359 nucleotide sequences described herein can be used to prepare two PCR primers from the 5' and 3' ends of the sequences. These primers can then be used to amplify an individual's DNA and subsequently sequence it. Panels of corresponding DNA sequences from individuals, prepared in this manner, can provide unique individual identifications, as each individual will have a unique set of such DNA sequences due to allelic differences.

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO: 1 can provide positive individual identification with a panel of perhaps 10 to 1,000 primers which each yield a noncoding amplified sequence of 100 bases. If predicted coding sequences, such as those in SEQ ID NO:3 are used, a more appropriate number of primers for positive individual identification would be 500-2,000.

If a panel of reagents from 98359 nucleotide sequences described herein is used to generate a unique identification database for an individual, those same reagents can later be used to identify tissue from that individual. Using the unique identification database, positive identification of the individual, living or dead, can be made from extremely small tissue samples.

### Use of Partial 98359 Sequences in Forensic Biology

DNA-based identification techniques can also be used in forensic biology. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1 (e.g., fragments derived from the noncoding regions of SEQ ID NO:1 having a length of at least 20 bases, preferably at least 30 bases) are particularly appropriate for this use.

The 98359 nucleotide sequences described herein can further be used to provide polynucleotide reagents, e.g., labeled or labelable probes which can be used in, for example, an *in situ* hybridization technique, to identify a specific tissue. This can be very useful in cases where a forensic pathologist is presented with a tissue of unknown origin. Panels of such 98359 probes can be used to identify tissue by species and/or by organ type.

In a similar fashion, these reagents, e.g., 98359 primers or probes can be used to screen tissue culture for contamination (i.e. screen for the presence of a mixture of different types of cells in a culture).

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 98359.

Such disorders include, e.g., a disorder associated with the misexpression of 98359 gene; a disorder of the muscular (e.g., neuromuscular) or central nervous system.

The method includes one or more of the following:

detecting, in a tissue of the subject, the presence or absence of a mutation which affects the expression of the 98359 gene, or detecting the presence or absence of a mutation in a region which controls the expression of the gene, e.g., a mutation in the 5' control region;

detecting, in a tissue of the subject, the presence or absence of a mutation which alters the structure of the 98359 gene;

detecting, in a tissue of the subject, the misexpression of the 98359 gene, at the mRNA level, e.g., detecting a non-wild type level of an mRNA;

detecting, in a tissue of the subject, the misexpression of the gene, at the protein level, e.g., detecting a non-wild type level of a 98359 polypeptide.

In preferred embodiments the method includes: ascertaining the existence of at least one of: a deletion of one or more nucleotides from the 98359 gene; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, or naturally occurring mutants thereof or 5' or 3' flanking sequences naturally associated with the 98359 gene; (ii) exposing the probe/primer to nucleic acid of the tissue; and detecting, by hybridization, e.g., *in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 98359 gene; the presence of a non-wild type splicing pattern of a messenger RNA transcript of the gene; or a non-wild type level of 98359.

Methods of the invention can be used prenatally or to determine if a subject's offspring will be at risk for a disorder.

In preferred embodiments the method includes determining the structure of a 98359 gene, an abnormal structure being indicative of risk for the disorder.

In preferred embodiments the method includes contacting a sample from the subject with an antibody to the 98359 protein or a nucleic acid, which hybridizes specifically with the gene. These and other embodiments are discussed below.

### Diagnostic and Prognostic Assays

The presence, level, or absence of 98359 protein or nucleic acid in a biological sample can be evaluated by obtaining a biological sample from a test subject and contacting the biological sample with a compound or an agent capable of detecting 98359 protein or nucleic acid (e.g., mRNA, genomic DNA) that encodes 98359 protein such that the presence of 98359 protein or nucleic acid is detected in the biological sample. The term "biological sample" includes tissues, cells and biological fluids isolated from a subject, as well as tissues, cells and fluids present within a subject. A preferred biological sample is serum. The level of expression of the 98359 gene can be measured in a number of ways, including, but not limited to: measuring the mRNA encoded by the 98359 genes; measuring the amount of protein encoded by the 98359 genes; or measuring the activity of the protein encoded by the 98359 genes.

The level of mRNA corresponding to the 98359 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 98359 nucleic acid, such as the nucleic acid of SEQ ID NO:1, or a portion thereof, such as an oligonucleotide of at least 7, 15, 30, 50, 100, 250 or 500 nucleotides in length and sufficient to specifically hybridize under stringent conditions to 98359 mRNA or genomic DNA. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array. A skilled artisan can adapt known mRNA detection methods for use in detecting the level of mRNA encoded by the 98359 genes.

The level of mRNA in a sample that is encoded by one of 98359 can be evaluated with nucleic acid amplification, e.g., by rtPCR (Mullis (1987) U.S. Patent No. 4,683,202), ligase chain reaction (Barany (1991) *Proc. Natl. Acad. Sci. USA* 88:189-193), self sustained sequence replication (Guatelli *et al*., (1990) *Proc. Natl. Acad. Sci. USA* 87:1874-1878), transcriptional amplification system (Kwoh *et al*., (1989), *Proc. Natl. Acad. Sci. USA* 86:1173-1177), Q-Beta Replicase (Lizardi *et al*., (1988) *Bio* /*Technology 6*:1197), rolling circle replication (Lizardi *et al*., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 98359 gene being analyzed.

In another embodiment, the methods further contacting a control sample with a compound or agent capable of detecting 98359 mRNA, or genomic DNA, and comparing the presence of 98359 mRNA or genomic DNA in the control sample with the presence of 98359 mRNA or genomic DNA in the test sample.

A variety of methods can be used to determine the level of protein encoded by 98359. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (*i.e*., physically linking) a detectable substance to the probe or antibody, as well as indirect labeling of the probe or antibody by reactivity with a detectable substance. Examples of detectable substances are provided herein.

The detection methods can be used to detect 98359 protein in a biological sample *in vitro* as well as *in vivo. In vitro* techniques for detection of 98359 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 98359 protein include introducing into a subject a labeled anti-98359 antibody. For example, the antibody can be labeled with a radioactive marker whose presence and location in a subject can be detected by standard imaging techniques.

In another embodiment, the methods further include contacting the control sample with a compound or agent capable of detecting 98359 protein, and comparing the presence of 98359 protein in the control sample with the presence of 98359 protein in the test sample.

The invention also includes kits for detecting the presence of 98359 in a biological sample. For example, the kit can include a compound or agent capable of detecting 98359 protein or mRNA in a biological sample; and a standard. The compound or agent can be packaged in a suitable container. The kit can further comprise instructions for using the kit to detect 98359 protein or nucleic acid.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 98359 expression or activity. As used herein, the term "unwanted" includes an unwanted phenomenon involved in a biological response such as pain or deregulated cell proliferation.

In one embodiment, a disease or disorder associated with aberrant or unwanted 98359 expression or activity is identified. A test sample is obtained from a subject and 98359 protein or nucleic acid (e.g., mRNA or genomic DNA) is evaluated, wherein the level, e.g., the presence or absence, of 98359 protein or nucleic acid is diagnostic for a subject having or at risk of developing a disease or disorder associated with aberrant or unwanted 98359 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 98359 expression or activity. For example, such methods can be used to determine whether a subject can be effectively treated with an agent for a muscular or central nervous system disorder.

The methods of the invention can also be used to detect genetic alterations in a 98359 gene, thereby determining if a subject with the altered gene is at risk for a disorder characterized by misregulation in 98359 protein activity or nucleic acid expression, such as a muscular or central nervous system disorder. In preferred embodiments, the methods include detecting, in a sample from the subject, the presence or absence of a genetic alteration characterized by at least one of an alteration affecting the integrity of a gene encoding a 98359-protein, or the mis-expression of the 98359 gene. For example, such genetic alterations can be detected by ascertaining the existence of at least one of 1) a deletion of one or more nucleotides from a 98359 gene; 2) an addition of one or more nucleotides to a 98359 gene; 3) a substitution of one or more nucleotides of a 98359 gene, 4) a chromosomal rearrangement of a 98359 gene; 5) an alteration in the level of a messenger RNA transcript of a 98359 gene, 6) aberrant modification of a 98359 gene, such as of the methylation pattern of the genomic DNA, 7) the presence of a non-wild type splicing pattern of a messenger RNA transcript of a 98359 gene, 8) a non-wild type level of a 98359-protein, 9) allelic loss of a 98359 gene, and 10) inappropriate post-translational modification of a 98359-protein.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 98359-gene. This method can include the steps of collecting a sample of cells from a subject, isolating nucleic acid (e.g., genomic, mRNA or both) from the sample, contacting the nucleic acid sample with one or more primers which specifically hybridize to a 98359 gene under conditions such that hybridization and amplification of the 98359 gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein. Alternatively, other amplification methods described herein or known in the art can be used.

In another embodiment, mutations in a 98359 gene from a sample cell can be identified by detecting alterations in restriction enzyme cleavage patterns. For example, sample and control DNA is isolated, amplified (optionally), digested with one or more restriction endonucleases, and fragment length sizes are determined, e.g., by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 98359 can be identified by hybridizing a sample and control nucleic acids, e.g., DNA or RNA, two dimensional arrays, e.g., chip based arrays. Such arrays include a plurality of addresses, each of which is positionally distinguishable from the other. A different probe is located at each address of the plurality. The arrays can have a high density of addresses, e.g., can contain hundreds or thousands of oligonucleotides probes (Cronin *et al*. (1996) *Human Mutation* 7: 244-255; Kozal *et al*. (1996) *Nature Medicine* 2: 753-759). For example, genetic mutations in 98359 can be identified in two dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 98359 gene and detect mutations by comparing the sequence of the sample 98359 with the corresponding wild-type (control) sequence. Automated sequencing procedures can be utilized when performing the diagnostic assays (Naeve *et al*. (1995) *Biotechniques* 19:448-53), including sequencing by mass spectrometry.

Other methods for detecting mutations in the 98359 gene include methods in which protection from cleavage agents is used to detect mismatched bases in RNA/RNA or RNA/DNA heteroduplexes (Myers *et al*. (1985) *Science* 230:1242; Cotton *et al*. (1988) *Proc. Natl Acad Sci USA* 85:4397; Saleeba *et al*. (1992) *Methods Enzymol*. 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 98359 cDNAs obtained from samples of cells. For example, the mutY enzyme of *E. coli* cleaves A at G/A mismatches and the thymidine DNA glycosylase from HeLa cells cleaves T at G/T mismatches (Hsu *et al*. (1994) *Carcinogenesis* 15:1657-1662; U.S. Patent No. 5,459,039).

In other embodiments, alterations in electrophoretic mobility will be used to identify mutations in 98359 genes. For example, single strand conformation polymorphism (SSCP) can be used to detect differences in electrophoretic mobility between mutant and wild type nucleic acids (Orita *et al*. (1989) *Proc Natl. Acad. Sci USA*: 86:2766, see also Cotton (1993) *Mutat. Res.* 285:125-144; and Hayashi (1992) *Genet. Anal. Tech. Appl.* 9:73-79). Single-stranded DNA fragments of sample and control 98359 nucleic acids will be denatured and allowed to renature. The secondary structure of single-stranded nucleic acids varies according to sequence, the resulting alteration in electrophoretic mobility enables the detection of even a single base change. The DNA fragments can be labeled or detected with labeled probes. The sensitivity of the assay can be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen *et al*. (1991) *Trends Genet* 7:5).

In yet another embodiment, the movement of mutant or wild-type fragments in polyacrylamide gels containing a gradient of denaturant is assayed using denaturing gradient gel electrophoresis (DGGE) (Myers *et al*. (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys Chem* 265:12753).

Examples of other techniques for detecting point mutations include, but are not limited to, selective oligonucleotide hybridization, selective amplification, or selective primer extension (Saiki *et al*. (1986) *Nature* 324:163); Saiki *et al*. (1989) *Proc. Natl Acad. Sci USA* 86:6230).

Alternatively, allele specific amplification technology which depends on selective PCR amplification can be used in conjunction with the instant invention. Oligonucleotides used as primers for specific amplification can carry the mutation of interest in the center of the molecule (so that amplification depends on differential hybridization) (Gibbs *et al*. (1989) *Nucleic Acids Res.* 17:2437-2448) or at the extreme 3' end of one primer where, under appropriate conditions, mismatch can prevent, or reduce polymerase extension (Prossner (1993) *Tibtech* 11:238). In addition it may be desirable to introduce a novel restriction site in the region of the mutation to create cleavage-based detection (Gasparini *et al*. (1992) *Mol. Cell Probes* 6:1). It is anticipated that in certain embodiments amplification can also be performed using Taq ligase for amplification (Barany (1991) *Proc. Natl. Acad. Sci USA* 88:189-93). In such cases, ligation will occur only if there is a perfect match at the 3' end of the 5' sequence making it possible to detect the presence of a known mutation at a specific site by looking for the presence or absence of amplification.

The methods described herein can be performed, for example, by utilizing pre-packaged diagnostic kits comprising at least one probe nucleic acid or antibody reagent described herein, which can be conveniently used, e.g., in clinical settings to diagnose patients exhibiting symptoms or family history of a disease or illness involving a 98359 gene.

### Use of 98359 Molecules as Surrogate Markers

The 98359 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the 98359 molecules of the invention can be detected, and can be correlated with one or more biological states *in vivo.* For example, the 98359 molecules of the invention can serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (e.g., with the presence or absence of a tumor). The presence or quantity of such markers is independent of the disease. Therefore, these markers can serve to indicate whether a particular course of treatment is effective in lessening a disease state or disorder. Surrogate markers are of particular use when the presence or extent of a disease state or disorder is difficult to assess through standard methodologies (e.g., early stage tumors), or when an assessment of disease progression is desired before a potentially dangerous clinical endpoint is reached (e.g., an assessment of cardiovascular disease can be made using cholesterol levels as a surrogate marker, and an analysis of HIV infection can be made using HIV RNA levels as a surrogate marker, well in advance of the undesirable clinical outcomes of myocardial infarction or fully-developed AIDS). Examples of the use of surrogate markers in the art include: Koomen *et al*. (2000) *J. Mass. Spectrom.* 35: 258-264; and James (1994) *AIDS Treatment News Archive* 209.

The 98359 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker can be indicative of the concentration of the drug in a biological tissue, in that the marker is either expressed or transcribed or not expressed or transcribed in that tissue in relationship to the level of the drug. In this fashion, the distribution or uptake of the drug can be monitored by the pharmacodynamic marker. Similarly, the presence or quantity of the pharmacodynamic marker can be related to the presence or quantity of the metabolic product of a drug, such that the presence or quantity of the marker is indicative of the relative breakdown rate of the drug *in vivo.* Pharmacodynamic markers are of particular use in increasing the sensitivity of detection of drug effects, particularly when the drug is administered in low doses. Since even a small amount of a drug can be sufficient to activate multiple rounds of marker (e.g., a 98359 marker) transcription or expression, the amplified marker can be in a quantity which is more readily detectable than the drug itself. Also, the marker can be more easily detected due to the nature of the marker itself; for example, using the methods described herein, anti-98359 antibodies can be employed in an immune-based detection system for a 98359 protein marker, or 98359-specific radiolabeled probes can be used to detect a 98359 mRNA marker. Furthermore, the use of a pharmacodynamic marker can offer mechanism-based prediction of risk due to drug treatment beyond the range of possible direct observations. Examples of the use of pharmacodynamic markers in the art include: Matsuda *et al*. US 6,033,862; Hattis *et al*. (1991) *Env. Health Perspect*. 90: 229-238; Schentag (1999) *Am. J. Health-Syst. Pharm*. 56 Suppl. 3: S21-S24; and Nicolau (1999) *Am. J. Health-Syst. Pharm.* 56 Suppl. 3: S16-S20.

The 98359 molecules of the invention are also useful as pharmacogenomic markers. As used herein, a "pharmacogenomic marker" is an objective biochemical marker which correlates with a specific clinical drug response or susceptibility in a subject (see, e.g., McLeod *et al*. (1999) *Eur. J. Cancer* 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, can be selected. For example, based on the presence or quantity of RNA, or protein (e.g., 98359 protein or RNA) for specific tumor markers in a subject, a drug or course of treatment can be selected that is optimized for the treatment of the specific tumor likely to be present in the subject. Similarly, the presence or absence of a specific sequence mutation in 98359 DNA can correlate with a 98359 drug response. The use of pharmacogenomic markers therefore permits the application of the most appropriate treatment for each subject without having to administer the therapy.

### Pharmaceutical Compositions

The nucleic acid and polypeptides, fragments thereof, as well as anti-98359 antibodies (also referred to herein as "active compounds") of the invention can be incorporated into pharmaceutical compositions. Such compositions typically include the nucleic acid molecule, protein, or antibody and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ ED₅₀. Compounds which exhibit high therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma can be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody, unconjugated or conjugated as described herein, can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank *et al*. ((1997) *J. Acquired Immune Deficiency Syndromes and Human Retrovirology* 14:193).

The present invention encompasses agents which modulate expression or activity. An agent can, for example, be a small molecule. For example, such small molecules include, but are not limited to, peptides, peptidomimetics (e.g., peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid of the invention, a physician, veterinarian, or researcher can, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen *et al*. (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Methods of Treatment:

The present invention provides for both prophylactic and therapeutic methods of treating a subject at risk of (or susceptible to) a disorder or having a disorder associated with aberrant or unwanted 98359 expression or activity. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides.

With regards to both prophylactic and therapeutic methods of treatment, such treatments can be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 98359 molecules of the present invention or 98359 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

In one aspect, the invention provides a method for preventing in a subject, a disease or condition associated with an aberrant or unwanted 98359 expression or activity, by administering to the subject a 98359 or an agent which modulates 98359 expression or at least one 98359 activity. Subjects at risk for a disease which is caused or contributed to by aberrant or unwanted 98359 expression or activity can be identified by, for example, any or a combination of diagnostic or prognostic assays as described herein. Administration of a prophylactic agent can occur prior to the manifestation of symptoms characteristic of the 98359 aberrance, such that a disease or disorder is prevented or, alternatively, delayed in its progression. Depending on the type of 98359 aberrance, for example, a 98359, 98359 agonist or 98359 antagonist agent can be used for treating the subject. The appropriate agent can be determined based on screening assays described herein.

It is possible that some 98359 disorders can be caused, at least in part, by an abnormal level of gene product, or by the presence of a gene product exhibiting abnormal activity. As such, the reduction in the level and/or activity of such gene products would bring about the amelioration of disorder symptoms.

Therefore, the 98359 molecules of the invention can act as novel diagnostic targets and therapeutic agents for controlling at least one or more disorders of the following types, as described herein: sodium channel disorders, neurophysiological disorders and complications, immune disorders, neurological disorders (including CNS disorders), muscular disorders, gastrointestinal disorders, mammary gland disorders, cardiovascular disorders, diaphragm disorders, skin disorders, and pain disorders.

Further examples of disorders which can be treated and/or diagnosed by the 98350 molecules of the invention include but are not limited to, disorders associated with viral diseases, bone metabolism, and liver.

Additionally, 98359 molecules can play an important role in the etiology of certain viral diseases, including but not limited to Hepatitis B, Hepatitis C and Herpes Simplex Virus (HSV). Modulators of 98359 activity could be used to control viral diseases. The modulators can be used in the treatment and/or diagnosis of viral infected tissue or virus-associated tissue fibrosis, especially liver and liver fibrosis. Also, 98359 modulators can be used in the treatment and/or diagnosis of virus-associated carcinoma, especially hepatocellular cancer.

Additionally, 98359 can play an important role in the regulation of disorders associated with bone metabolism. "Bone metabolism" refers to direct or indirect effects in the formation or degeneration of bone structures, e.g., bone formation, bone resorption, etc., which can ultimately affect the concentrations in serum of calcium and phosphate. This term also includes activities mediated by 98359 molecules in bone cells, e.g., osteoclasts and osteoblasts, that can in turn result in bone formation and degeneration. For example, 98359 molecules can support different activities of bone resorbing osteoclasts such as the stimulation of differentiation of monocytes and mononuclear phagocytes into osteoclasts. Accordingly, 98359 molecules that modulate the production of bone cells can influence bone formation and degeneration, and thus can be used to treat bone disorders. Examples of such disorders include, but are not limited to, osteoporosis, osteodystrophy, osteomalacia, rickets, osteitis fibrosa cystica, renal osteodystrophy, osteosclerosis, anti-convulsant treatment, osteopenia, fibrogenesis-imperfecta ossium, secondary hyperparathyrodism, hypoparathyroidism, hyperparathyroidism, cirrhosis, obstructive jaundice, drug induced metabolism, medullary carcinoma, chronic renal disease, rickets, sarcoidosis, glucocorticoid antagonism, malabsorption syndrome, steatorrhea, tropical sprue, idiopathic hypercalcemia and milk fever.

Additionally, 98359 can play an important role in the regulation of disorders associated with the liver. Disorders which can be treated or diagnosed by methods described herein include, but are not limited to, disorders associated with an accumulation in the liver of fibrous tissue, such as that resulting from an imbalance between production and degradation of the extracellular matrix accompanied by the collapse and condensation of preexisting fibers. The methods described herein can be used to diagnose or treat hepatocellular necrosis or injury induced by a wide variety of agents including processes which disturb homeostasis, such as an inflammatory process, tissue damage resulting from toxic injury or altered hepatic blood flow, and infections (e.g., bacterial, viral and parasitic). For example, the methods can be used for the early detection of hepatic injury, such as portal hypertension or hepatic fibrosis. In addition, the methods can be employed to detect liver fibrosis attributed to inborn errors of metabolism, for example, fibrosis resulting from a storage disorder such as Gaucher's disease (lipid abnormalities) or a glycogen storage disease, Al-antitrypsin deficiency; a disorder mediating the accumulation (e.g., storage) of an exogenous substance, for example, hemochromatosis (iron-overload syndrome) and copper storage diseases (Wilson's disease), disorders resulting in the accumulation of a toxic metabolite (e.g., tyrosinemia, fructosemia and galactosemia) and peroxisomal disorders (e.g., Zellweger syndrome). Additionally, the methods described herein can be used for the early detection and treatment of liver injury associated with the administration of various chemicals or drugs, such as for example, methotrexate, isonizaid, oxyphenisatin, methyldopa, chlorpromazine, tolbutamide or alcohol, or which represents a hepatic manifestation of a vascular disorder such as obstruction of either the intrahepatic or extrahepatic bile flow or an alteration in hepatic circulation resulting, for example, from chronic heart failure, veno-occlusive disease, portal vein thrombosis or Budd-Chiari syndrome.

As discussed, successful treatment of 98359 disorders can be brought about by techniques that serve to inhibit the expression or activity of target gene products. For example, compounds, e.g., an agent identified using an assays described above, that proves to exhibit negative modulatory activity, can be used in accordance with the invention to prevent and/or ameliorate symptoms of 98359 disorders. Such molecules can include, but are not limited to peptides, phosphopeptides, small organic or inorganic molecules, or antibodies (including, for example, polyclonal, monoclonal, humanized, human, anti-idiotypic, chimeric or single chain antibodies, and Fab, F(ab')₂ and Fab expression library fragments, scFV molecules, and epitope-binding fragments thereof).

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to co-administer normal target gene protein into the cell or tissue in order to maintain the requisite level of cellular or tissue target gene activity.

Another method by which nucleic acid molecules can be utilized in treating or preventing a disease characterized by 98359 expression is through the use of aptamer molecules specific for 98359 protein. Aptamers are nucleic acid molecules having a tertiary structure which permits them to specifically or selectively bind to protein ligands (see, e.g., Osborne *et al*. (1997) *Curr. Opin. Chem Biol.* 1: 5-9; and Patel (1997) *Curr Opin Chem Biol* 1:32-46). Since nucleic acid molecules can in many cases be more conveniently introduced into target cells than therapeutic protein molecules can be, aptamers offer a method by which 98359 protein activity can be specifically decreased without the introduction of drugs or other molecules which can have pluripotent effects.

Antibodies can be generated that are both specific for target gene product and that reduce target gene product activity. Such antibodies can, therefore, by administered in instances whereby negative modulatory techniques are appropriate for the treatment of 98359 disorders. For a description of antibodies, see the Antibody section above.

In circumstances wherein injection of an animal or a human subject with a 98359 protein or epitope for stimulating antibody production is harmful to the subject, it is possible to generate an immune response against 98359 through the use of anti-idiotypic antibodies (see, for example, Herlyn (1999) *Ann Med* 31:66-78; and Bhattacharya-Chatterjee and Foon (1998) *Cancer Treat Res.* 94:51-68). If an anti-idiotypic antibody is introduced into a mammal or human subject, it should stimulate the production of anti-anti-idiotypic antibodies, which should be specific to the 98359 protein. Vaccines directed to a disease characterized by 98359 expression can also be generated in this fashion.

In instances where the target antigen is intracellular and whole antibodies are used, internalizing antibodies can be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see e.g., Marasco *et al*. (1993) *Proc. Natl. Acad. Sci. USA* 90:7889-7893).

The identified compounds that inhibit target gene expression, synthesis and/or activity can be administered to a patient at therapeutically effective doses to prevent, treat or ameliorate 98359 disorders. A therapeutically effective dose refers to that amount of the compound sufficient to result in amelioration of symptoms of the disorders. Toxicity and therapeutic efficacy of such compounds can be determined by standard pharmaceutical procedures as described above.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of determination of effective dose for an individual is directly assay levels of "free" and "bound" compound in the serum of the test subject. Such assays can utilize antibody mimics and/or "biosensors" that have been created through molecular imprinting techniques. The compound which is able to modulate 98359 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell *et al* (1996) *Current Opinion in Biotechnology* 7:89-94 and in Shea (1994) *Trends in Polymer Science* 2:166-173. Such "imprinted" affinity matrixes are amenable to ligand-binding assays, whereby the immobilized monoclonal antibody component is replaced by an appropriately imprinted matrix. An example of the use of such matrixes in this way can be seen in Vlatakis *et al* (1993) *Nature* 361:645-647. Through the use of isotope-labeling, the "free" concentration of compound which modulates the expression or activity of 98359 can be readily monitored and used in calculations of IC₅₀.

Such "imprinted" affinity matrixes can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. An rudimentary example of such a "biosensor" is discussed in Kriz *et al* (1995) *Analytical Chemistry* 67:2142-2144.

Another aspect of the invention pertains to methods of modulating 98359 expression or activity for therapeutic purposes. Accordingly, in an exemplary embodiment, the modulatory method of the invention involves contacting a cell with a 98359 or agent that modulates one or more of the activities of 98359 protein activity associated with the cell. An agent that modulates 98359 protein activity can be an agent as described herein, such as a nucleic acid or a protein, a naturally-occurring target molecule of a 98359 protein (e.g., a 98359 substrate or receptor), a 98359 antibody, a 98359 agonist or antagonist, a peptidomimetic of a 98359 agonist or antagonist, or other small molecule.

In one embodiment, the agent stimulates one or 98359 activities. Examples of such stimulatory agents include active 98359 protein and a nucleic acid molecule encoding 98359. In another embodiment, the agent inhibits one or more 98359 activities. Examples of such inhibitory agents include antisense 98359 nucleic acid molecules, anti-98359 antibodies, and 98359 inhibitors. These modulatory methods can be performed *in vitro* (e.g., by culturing the cell with the agent) or, alternatively, *in vivo* (e.g., by administering the agent to a subject). As such, the present invention provides methods of treating an individual afflicted with a disease or disorder characterized by aberrant or unwanted expression or activity of a 98359 protein or nucleic acid molecule. In one embodiment, the method involves administering an agent (e.g., an agent identified by a screening assay described herein), or combination of agents that modulates (e.g., up regulates or down regulates) 98359 expression or activity. In another embodiment, the method involves administering a 98359 protein or nucleic acid molecule as therapy to compensate for reduced, aberrant, or unwanted 98359 expression or activity.

Stimulation of 98359 activity is desirable in situations in which 98359 is abnormally downregulated and/or in which increased 98359 activity is likely to have a beneficial effect. For example, stimulation of 98359 activity is desirable in situations in which a 98359 is downregulated and/or in which increased 98359 activity is likely to have a beneficial effect. Likewise, inhibition of 98359 activity is desirable in situations in which 98359 is abnormally upregulated and/or in which decreased 98359 activity is likely to have a beneficial effect.

### Pharmacogenomics

The 98359 molecules of the present invention, as well as agents, or modulators which have a stimulatory or inhibitory effect on 98359 activity (e.g., 98359 gene expression) as identified by a screening assay described herein can be administered to individuals to treat (prophylactically or therapeutically) 98359-associated disorders (e.g., aberrant or deficient muscular (e.g., neuromuscular) or central nervous system-related disorder) associated with aberrant or unwanted 98359 activity. In conjunction with such treatment, pharmacogenomics (i.e., the study of the relationship between an individual's genotype and that individual's response to a foreign compound or drug) can be considered. Differences in metabolism of therapeutics can lead to severe toxicity or therapeutic failure by altering the relation between dose and blood concentration of the pharmacologically active drug. Thus, a physician or clinician can consider applying knowledge obtained in relevant pharmacogenomics studies in determining whether to administer a 98359 molecule or 98359 modulator as well as tailoring the dosage and/or therapeutic regimen of treatment with a 98359 molecule or 98359 modulator.

Pharmacogenomics deals with clinically significant hereditary variations in the response to drugs due to altered drug disposition and abnormal action in affected persons. See, for example, Eichelbaum *et al*. (1996) *Clin. Exp. Pharmacol. Physiol.* 23:983-985 and Linder *et al.* (1997) *Clin. Chem.* 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP can occur once per every 1000 bases of DNA. A SNP can be involved in a disease process, however, the vast majority can not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that can be common among such genetically similar individuals.

Alternatively, a method termed the "candidate gene approach", can be utilized to identify genes that predict drug response. According to this method, if a gene that encodes a drug's target is known (e.g., a 98359 protein of the present invention), all common variants of that gene can be fairly easily identified in the population and it can be determined if having one version of the gene versus another is associated with a particular drug response.

Alternatively, a method termed the "gene expression profiling", can be utilized to identify genes that predict drug response. For example, the gene expression of an animal dosed with a drug (e.g., a 98359 molecule or 98359 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 98359 molecule or 98359 modulator, such as a modulator identified by one of the exemplary screening assays described herein.

The present invention further provides methods for identifying new agents, or combinations, that are based on identifying agents that modulate the activity of one or more of the gene products encoded by one or more of the 98359 genes of the present invention, wherein these products can be associated with resistance of the cells to a therapeutic agent. Specifically, the activity of the proteins encoded by the 98359 genes of the present invention can be used as a basis for identifying agents for overcoming agent resistance. By blocking the activity of one or more of the resistance proteins, target cells, e.g., human cells, will become sensitive to treatment with an agent to which the unmodified target cells were resistant.

Monitoring the influence of agents (e.g., drugs) on the expression or activity of a 98359 protein can be applied in clinical trials. For example, the effectiveness of an agent determined by a screening assay as described herein to increase 98359 gene expression, protein levels, or upregulate 98359 activity, can be monitored in clinical trials of subjects exhibiting decreased 98359 gene expression, protein levels, or downregulated 98359 activity. Alternatively, the effectiveness of an agent determined by a screening assay to decrease 98359 gene expression, protein levels, or downregulate 98359 activity, can be monitored in clinical trials of subjects exhibiting increased 98359 gene expression, protein levels, or upregulated 98359 activity. In such clinical trials, the expression or activity of a 98359 gene, and preferably, other genes that have been implicated in, for example, a sodium channel beta-4 subunit-associated or another 98359-associated disorder can be used as a "read out" or markers of the phenotype of a particular cell.

### Other Embodiments

In another aspect, the invention features a method of analyzing a plurality of capture probes. The method is useful, e.g., to analyze gene expression. The method includes: providing a two dimensional array having a plurality of addresses, each address of the plurality being positionally distinguishable from each other address of the plurality, and each address of the plurality having a unique capture probe, e.g., a nucleic acid or peptide sequence, wherein the capture probes are from a cell or subject which expresses 98359 or from a cell or subject in which a 98359 mediated response has been elicited; contacting the array with a 98359 nucleic acid (preferably purified), a 98359 polypeptide (preferably purified), or an anti-98359 antibody, and thereby evaluating the plurality of capture probes. Binding, e.g., in the case of a nucleic acid, hybridization with a capture probe at an address of the plurality, is detected, e.g., by a signal generated from a label attached to the 98359 nucleic acid, polypeptide, or antibody.

The capture probes can be a set of nucleic acids from a selected sample, e.g., a sample of nucleic acids derived from a control or non-stimulated tissue or cell.

The method can include contacting the 98359 nucleic acid, polypeptide, or antibody with a first array having a plurality of capture probes and a second array having a different plurality of capture probes. The results of each hybridization can be compared, e.g., to analyze differences in expression between a first and second sample. The first plurality of capture probes can be from a control sample, e.g., a wild type, normal, or non-diseased, non-stimulated, sample, e.g., a biological fluid, tissue, or cell sample. The second plurality of capture probes can be from an experimental sample, e.g., a mutant type, at risk, disease-state or disorder-state, or stimulated, sample, e.g., a biological fluid, tissue, or cell sample.

The plurality of capture probes can be a plurality of nucleic acid probes each of which specifically hybridizes, with an allele of 98359. Such methods can be used to diagnose a subject, e.g., to evaluate risk for a disease or disorder, to evaluate suitability of a selected treatment for a subject, to evaluate whether a subject has a disease or disorder.

The method can be used to detect SNPs, as described above.

In another aspect, the invention features, a method of analyzing 98359, e.g., analyzing structure, function, or relatedness to other nucleic acid or amino acid sequences. The method includes: providing a 98359 nucleic acid or amino acid sequence; comparing the 98359 sequence with one or more preferably a plurality of sequences from a collection of sequences, e.g., a nucleic acid or protein sequence database; to thereby analyze 98359.

The method can include evaluating the sequence identity between a 98359 sequence and a database sequence. The method can be performed by accessing the database at a second site, e.g., over the internet. Preferred databases include GenBank™ and SwissProt.

In another aspect, the invention features, a set of oligonucleotides, useful, e.g., for identifying SNP's, or identifying specific alleles of 98359. The set includes a plurality of oligonucleotides, each of which has a different nucleotide at an interrogation position, e.g., an SNP or the site of a mutation. In a preferred embodiment, the oligonucleotides of the plurality identical in sequence with one another (except for differences in length). The oligonucleotides can be provided with differential labels, such that an oligonucleotide which hybridizes to one allele provides a signal that is distinguishable from an oligonucleotides which hybridizes to a second allele.

The sequences of 98359 molecules are provided in a variety of mediums to facilitate use thereof. A sequence can be provided as a manufacture, other than an isolated nucleic acid or amino acid molecule, which contains a 98359 molecule. Such a manufacture can provide a nucleotide or amino acid sequence, e.g., an open reading frame, in a form which allows examination of the manufacture using means not directly applicable to examining the nucleotide or amino acid sequences, or a subset thereof, as they exist in nature or in purified form.

A 98359 nucleotide or amino acid sequence can be recorded on computer readable media. As used herein, "computer readable media" refers to any medium that can be read and accessed directly by a computer. Such media include, but are not limited to: magnetic storage media, such as floppy discs, hard disc storage medium, and magnetic tape; optical storage media such as compact disc and CD-ROM; electrical storage media such as RAM, ROM, EPROM, EEPROM, and the like; and general hard disks and hybrids of these categories such as magnetic/optical storage media. The medium is adapted or configured for having thereon 98359 sequence information of the present invention.

As used herein, the term "electronic apparatus" is intended to include any suitable computing or processing apparatus of other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phones, pagers, and the like; and local and distributed processing systems.

As used herein, "recorded" refers to a process for storing or encoding information on the electronic apparatus readable medium. Those skilled in the art can readily adopt any of the presently known methods for recording information on known media to generate manufactures comprising the 98359 sequence information.

A variety of data storage structures are available to a skilled artisan for creating a computer readable medium having recorded thereon a 98359 nucleotide or amino acid sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. The skilled artisan can readily adapt any number of data processor structuring formats (e.g., text file or database) in order to obtain computer readable medium having recorded thereon the nucleotide sequence information of the present invention.

By providing the 98359 nucleotide or amino acid sequences of the invention in computer readable form, the skilled artisan can routinely access the sequence information for a variety of purposes. For example, one skilled in the art can use the nucleotide or amino acid sequences of the invention in computer readable form to compare a target sequence or target structural motif with the sequence information stored within the data storage means. A search is used to identify fragments or regions of the sequences of the invention which match a particular target sequence or target motif.

The present invention therefore provides a medium for holding instructions for performing a method for determining whether a subject has a sodium channel beta-4 subunit-associated or another 98359-associated disease or disorder or a pre-disposition to a sodium channel beta-4 subunit-associated or another 98359-associated disease or disorder, wherein the method comprises the steps of determining 98359 sequence information associated with the subject and based on the 98359 sequence information, determining whether the subject has a sodium channel beta-4 subunit-associated or another 98359-associated disease or disorder and/or recommending a particular treatment for the disease, disorder, or pre-disease condition.

The present invention further provides in an electronic system and/or in a network, a method for determining whether a subject has a sodium channel beta-4 subunit-associated or another 98359-associated disease or disorder or a pre-disposition to a disease associated with 98359, wherein the method comprises the steps of determining 98359 sequence information associated with the subject, and based on the 98359 sequence information, determining whether the subject has a sodium channel beta-4 subunit-associated or another 98359-associated disease or disorder or a pre-disposition to a sodium channel beta-4 subunit-associated or another 98359-associated disease or disorder, and/or recommending a particular treatment for the disease, disorder, or pre-disease condition. The method may further comprise the step of receiving phenotypic information associated with the subject and/or acquiring from a network phenotypic information associated with the subject.

The present invention also provides in a network, a method for determining whether a subject has a sodium channel beta-4 subunit-associated or another 98359-associated disease or disorder or a pre-disposition to a sodium channel beta-4 subunit-associated or another 98359-associated disease or disorder, said method comprising the steps of receiving 98359 sequence information from the subject and/or information related thereto, receiving phenotypic information associated with the subject, acquiring information from the network corresponding to 98359 and/or corresponding to a sodium channel beta-4 subunit-associated or another 98359-associated disease or disorder, and based on one or more of the phenotypic information, the 98359 information (e.g., sequence information and/or information related thereto), and the acquired information, determining whether the subject has a sodium channel beta-4 subunit-associated or another 98359-associated disease or disorder or a pre-disposition to a sodium channel beta-4 subunit-associated or another 98359-associated disease or disorder. The method may further comprise the step of recommending a particular treatment for the disease, disorder, or pre-disease condition.

The present invention also provides a business method for determining whether a subject has a sodium channel beta-4 subunit-associated or another 98359-associated disease or disorder or a pre-disposition to a sodium channel beta-4 subunit-associated or another 98359-associated disease or disorder, said method comprising the steps of receiving information related to 98359 (e.g., sequence information and/or information related thereto), receiving phenotypic information associated with the subject, acquiring information from the network related to 98359 and/or related to a sodium channel beta-4 subunit-associated or another 98359-associated disease or disorder, and based on one or more of the phenotypic information, the 98359 information, and the acquired information, determining whether the subject has a sodium channel beta-4 subunit-associated or another 98359-associated disease or disorder or a pre-disposition to a sodium channel beta-4 subunit-associated or another 98359-associated disease or disorder. The method may further comprise the step of recommending a particular treatment for the disease, disorder, or pre-disease condition.

The invention also includes an array comprising a 98359 sequence of the present invention. The array can be used to assay expression of one or more genes in the array. In one embodiment, the array can be used to assay gene expression in a tissue to ascertain tissue specificity of genes in the array. In this manner, up to about 7600 genes can be simultaneously assayed for expression, one of which can be 98359. This allows a profile to be developed showing a battery of genes specifically expressed in one or more tissues.

In addition to such qualitative information, the invention allows the quantitation of gene expression. Thus, not only tissue specificity, but also the level of expression of a battery of genes in the tissue if ascertainable. Thus, genes can be grouped on the basis of their tissue expression *per se* and level of expression in that tissue. This is useful, for example, in ascertaining the relationship of gene expression in that tissue. Thus, one tissue can be perturbed and the effect on gene expression in a second tissue can be determined. In this context, the effect of one cell type on another cell type in response to a biological stimulus can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more genes in the array. This can occur in various biological contexts, as disclosed herein, for example development of a sodium channel beta-4 subunit-associated or another 98359-associated disease or disorder, progression of sodium channel beta-4 subunit-associated or another 98359-associated disease or disorder, and processes, such a cellular transformation associated with the sodium channel beta-4 subunit-associated or another 98359-associated disease or disorder.

The array is also useful for ascertaining the effect of the expression of a gene on the expression of other genes in the same cell or in different cells (e.g., acertaining the effect of 98359 expression on the expression of other genes). This provides, for example, for a selection of alternate molecular targets for therapeutic intervention if the ultimate or downstream target cannot be regulated.

The array is also useful for ascertaining differential expression patterns of one or more genes in normal and abnormal cells. This provides a battery of genes (e.g., including 98359) that could serve as a molecular target for diagnosis or therapeutic intervention.

As used herein, a "target sequence" can be any DNA or amino acid sequence of six or more nucleotides or two or more amino acids. A skilled artisan can readily recognize that the longer a target sequence is, the less likely a target sequence will be present as a random occurrence in the database. Typical sequence lengths of a target sequence are from about 10 to 100 amino acids or from about 30 to 300 nucleotide residues. However, it is well recognized that commercially important fragments, such as sequence fragments involved in gene expression and protein processing, may be of shorter length.

Computer software is publicly available which allows a skilled artisan to access sequence information provided in a computer readable medium for analysis and comparison to other sequences. A variety of known algorithms are disclosed publicly and a variety of commercially available software for conducting search means are and can be used in the computer-based systems of the present invention. Examples of such software include, but are not limited to, MacPattern (EMBL), BLASTN and BLASTX (NCBI).

Thus, the invention features a method of making a computer readable record of a sequence of a 98359 sequence which includes recording the sequence on a computer readable matrix. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention features a method of analyzing a sequence. The method includes: providing a 98359 sequence, or record, in computer readable form; comparing a second sequence to the 98359 sequence; thereby analyzing a sequence. Comparison can include comparing to sequences for sequence identity or determining if one sequence is included within the other, e.g., determining if the 98359 sequence includes a sequence being compared. In a preferred embodiment the 98359 or second sequence is stored on a first computer, e.g., at a first site and the comparison is performed, read, or recorded on a second computer, e.g., at a second site. E.g., the 98359 or second sequence can be stored in a public or proprietary database in one computer, and the results of the comparison performed, read, or recorded on a second computer. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

This invention is further illustrated by the following exemplification, which should not be construed as limiting.

### EXAMPLES

### I. Gene Expression Analysis

Total RNA was prepared from various human tissues by a single step extraction method using RNA STAT-60 according to the manufacturer's instructions (TelTest, Inc). Each RNA preparation was treated with DNase I (Ambion) at 37°C for 1 hour. DNAse I treatment was determined to be complete if the sample required at least 38 PCR amplification cycles to reach a threshold level of fluorescence using beta--2 microglobulin as an internal amplicon reference. The integrity of the RNA samples following DNase I treatment was confirmed by agarose gel electrophoresis and ethidium bromide staining. After phenol extraction cDNA was prepared from the sample using the SUPERSCRIPT™ Choice System following the manufacturer's instructions (GibcoBRL). A negative control of RNA without reverse transcriptase was mock reverse transcribed for each RNA sample.

Human and rat 98359 expression was measured by TaqMan® quantitative PCR (Perkin Elmer Applied Biosystems) in cDNA prepared from a variety of normal and diseased (e.g., cancerous) human and rat tissues or cell lines.

Probes were designed by PrimerExpress software (PE Biosystems) based on the sequence of the human 98359 gene. Each human 98359 gene probe was labeled using FAM (6-carboxyfluorescein), and the beta-2-microglobulin reference probe was labeled with a different fluorescent dye, VIC. The differential labeling of the target gene and internal reference gene thus enabled measurement in same well. Forward and reverse primers and the probes for both beta-2-microglobulin and target gene were added to the TaqMan® Universal PCR Master Mix (PE Applied Biosystems). Although the final concentration of primer and probe could vary, each was internally consistent within a given experiment. A typical experiment contained 200nM of forward and reverse primers plus 100nM probe for beta--2 microglobulin and 600 nM forward and reverse primers plus 200 nM probe for the target gene. TaqMan matrix experiments were carried out on an ABI PRISM 7700 Sequence Detection System (PE Applied Biosystems). The thermal cycler conditions were as follows: hold for 2 min at 50°C and 10 min at 95°C, followed by two-step PCR for 40 cycles of 95°C for 15 sec followed by 60°C for 1 min.

The following method was used to quantitatively calculate human 98359 gene expression in the various tissues relative to beta--2 microglobulin expression in the same tissue. The threshold cycle (Ct) value is defined as the cycle at which a statistically significant increase in fluorescence is detected. A lower Ct value is indicative of a higher mRNA concentration. The Ct value of the human 98359 gene is normalized by subtracting the Ct value of the beta--2 microglobulin gene to obtain a _{Δ}Ct value using the following formula: _{Δ}Ct=Ct_{human 59914 and 59921} - Ct _{beta--2 microglobulin}. Expression is then calibrated against a cDNA sample showing a comparatively low level of expression of the human 98359 gene. The _{Δ}Ct value for the calibrator sample is then subtracted from _{Δ}Ct for each tissue sample according to the following formula: _{ΔΔ}Ct=_{Δ}Ct-ₛₐₘₚₗₑ - _{Δ}Ct-_{calibrator}. Relative expression is then calculated using the arithmetic formula given by 2^{-ΔΔCt}. Expression of the target human 98359 gene in each of the tissues tested is then graphically represented.

The results indicate 98359 expression pattern as follows: expression in at least the following human tissues and cell lines (described below in the Exemplification): high levels in brain, dorsal root ganglia, and muscle (including gastrointestinal muscle, specifically); and lower levels in heart, spinal cord, and mammary glands; and

expression in at least the following rat tissues and cell lines (described below in the Exemplification): high levels in dorsal root ganglia; moderate levels in brain, spinal cord, heart, diaphragm. and muscle (including gastrointestinal muscle, specifically); and lower levels in skin (e.g., hairy skin).

### II. Effect of β4 subunits on the voltage-dependence of activation and inactivation of sodium channels

Effect of β4 subunits on the voltage-dependence of activation and inactivation of sodium channels was tested. Conductance was determined from peak sodium currents, I, according to: G=I/(V-Vᵣₑᵥ) where V is the test pulse voltage and Vᵣₑᵥ is the measured reversal potential. Mean voltage dependence of inactivation was determined using current-voltage relationships assessed using individual transformed cells.

Sodium channel activation is shifted negatively by coexpression of the β4 subunit. Inactivation curves were determined using prepulses to potentials from -90 to 40 mV in 5 mV steps followed by test pulses to 0 mV. The following values of V_{1/2} (half inactivation voltage) and k (slope factor) were determined for activation and inactivation of rat brain type IIA sodium channel α subunits alone, with and without the addition of β4 subunit 98359

V_{1/2} activation: rIIA is -4.4 +/- 0.6 (mV, mean +/- SE). rIIA and β4 is 11.3 +/- 1.1.

K activation: rIIA is 6.4 +/- 0.2. rIIA and β4 is 6.6 +/- 0.3.

V_{1/2} inactivation: rIIA is -35.7 +/- 0.8. rIIA and β4 is -44.0 +/- 0.8.

K inactivation: rIIA is 9.0 +/- 0.5. rIIA and β4 is 7.6 +/- 0.6.

### III. Co-immunoprecipitation of β4 subunits

Co-immunoprecipitation of β4 subunits with the sodium channel Naᵥ1.2 α subunit was measured. TsA-201 cells were cotransfected with HA-tagged rNaᵥ1.2 α subunits and HA-tagged β4 subunits or with untagged rNaᵥ 1.2 α subunits and HA-tagged β4 subunits. Channels were immunoprecipitated with SP20 recognizing the sodium channel α subunit or with non-immune IgG. Immunoprecipitated proteins were separated on by SDS-PAGE under reducing conditions and transferred to nitrocellulose. Immunoblots were probed with anti-HA11, and co-immunoprecipitation of the α and β4 subunits was detected.

The contents of all references, patents and published patent applications cited throughout this application are incorporated herein by reference.

### Equivalents

Those skilled in the art will recognize, or be able to ascertain using no more than routine experimentation, many equivalents to the specific embodiments of the invention described herein.

## Claims

1. An isolated nucleic acid molecule selected from the group consisting of:
a) a nucleic acid molecule having a nucleotide sequence which is at least 90% identical to the nucleotide sequence of SEQ ID NO: 1, 3, or a complement thereof;
b) a nucleic acid molecule comprising at least 1800 nucleotide residues and having a nucleotide sequence identical to at least 1800 consecutive nucleotide residues of SEQ ID NO:1, 3, or a complement thereof;
c) a nucleic acid molecule which encodes a polypeptide comprising the amino acid sequence of SEQ ID NO:2;
d) a nucleic acid molecule which encodes a fragment of a polypeptide comprising the amino acid sequence of SEQ ID NO:2, wherein the fragment comprises at least 8 consecutive amino acid residues of SEQ ID NO:2; and
e) a nucleic acid molecule which encodes a naturally occurring allelic variant of a polypeptide comprising the amino acid sequence of SEQ ID NO:2, wherein the nucleic acid molecule hybridizes to a nucleic acid molecule consisting of the nucleotide sequence of SEQ ID NO:1, 3, or a complement thereof under stringent conditions.

2. The isolated nucleic acid molecule of claim 1, which is selected from the group consisting of:
a) a nucleic acid having the nucleotide sequence of SEQ ID NO:1, 3, or a complement thereof; and
b) a nucleic acid molecule which encodes a polypeptide having the amino acid sequence of SEQ ID NO:2.

3. The nucleic acid molecule of claim 1 or 2, further comprising vector nucleic acid sequences.

4. The nucleic acid molecule of claim 1, 2 or 3, further comprising nucleic acid sequences encoding a heterologous polypeptide.

5. A host cell which contains the nucleic acid molecule of any of claims 1 to 4.

6. The host cell of claim 5 which is a mammalian host cell.

7. A non-human mammalian host cell containing the nucleic acid molecule of any of claims 1 to 4.

8. An isolated polypeptide selected from the group consisting of:
a) a fragment of a polypeptide comprising the amino acid sequence of SEQ ID NO:2, wherein the fragment comprises at least 8 contiguous amino acids of SEQ ID NO:2;
b) a naturally occurring allelic variant of a polypeptide comprising the amino acid sequence of SEQ ID NO:2, wherein the polypeptide is encoded by a nucleic acid molecule which hybridizes to a nucleic acid molecule consisting of the nucleotide sequence of SEQ ID NO:1, 3, or a complement thereof under stringent conditions; and
c) a polypeptide which is encoded by a nucleic acid molecule comprising a nucleotide sequence which is at least 90% identical to a nucleic acid consisting of the nucleotide sequence of SEQ ID NO:2, or a complement thereof.

9. The isolated polypeptide of claim 8 having the amino acid sequence of SEQ ID NO:2.

10. The polypeptide of claim 8 or 9, wherein the amino acid sequence of the polypeptide further comprises heterologous amino acid residues.

11. An antibody which selectively binds with the polypeptide of claim 8, 9 or 10.

12. A method for producing a polypeptide selected from the group consisting of:
a) a polypeptide comprising the amino acid sequence of SEQ ID NO:2;
b) a polypeptide comprising a fragment of the amino acid sequence of SEQ ID NO:2, wherein the fragment comprises at least 8 contiguous amino acids of SEQ ID NO:2; and
c) a naturally occurring allelic variant of a polypeptide comprising the amino acid sequence of SEQ ID NO:2, wherein the polypeptide is encoded by a nucleic acid molecule which hybridizes to a nucleic acid molecule consisting of the nucleotide sequence of SEQ ID NO:1, 3, or a complement thereof under stringent conditions;
the method comprising culturing the host cell of claim 5, 6 or 7 under conditions in which the nucleic acid molecule is expressed.

13. A method for detecting the presence of a polypeptide of claim 8, 9 or 10 in a sample, comprising:
a) contacting the sample with a compound which selectively binds with a polypeptide of claim 8, 9 or 10, as the case may be; and
b) determining whether the compound binds with the polypeptide in the sample.

14. The method of claim 13, wherein the compound which binds with the polypeptide is an antibody.

15. A kit comprising a compound which selectively binds with a polypeptide of claim 8, 9 or 10 and instructions for use.

16. A method for detecting the presence of a nucleic acid molecule of any of claims 1 to 4 in a sample, comprising the steps of:
a) contacting the sample with a nucleic acid probe or primer which selectively hybridizes with the nucleic acid molecule; and
b) determining whether the nucleic acid probe or primer binds with a nucleic acid molecule in the sample.

17. The method of claim 16, wherein the sample comprises mRNA molecules and is contacted with a nucleic acid probe.

18. A kit comprising a compound which selectively hybridizes with a nucleic acid molecule of any of claims 1 to 4 and instructions for use.

19. A method for identifying a compound which binds with a polypeptide of claim 8, 9 or 10, the method comprising the steps of:
a) contacting a polypeptide, or a cell expressing a polypeptide of claim 8, 9 or 10, as the case may be with a test compound; and
b) determining whether the polypeptide binds with the test compound.

20. The method of claim 19, wherein the binding of the test compound with the polypeptide is detected by a method selected from the group consisting of:
a) detection of binding by direct detecting of test compound/polypeptide binding;
b) detection of binding using a competition binding assay;
c) detection of binding using an assay for sodium ion transmembrane transport.

21. A method for modulating the activity of a polypeptide of claim 8, 9 or 10 comprising contacting the polypeptide or a cell expressing the polypeptide of claim 8, 9 or 10, as the case may be with a compound which binds with the polypeptide in a sufficient concentration to modulate the activity of the polypeptide.

22. A method for identifying a compound which modulates the activity of a polypeptide of claim 8, 9 or 10 comprising:
a) contacting the polypeptide of claim 8, 9 or 10, as the case may be with a test compound; and
b) determining the effect of the test compound on the activity of the polypeptide to thereby identify a compound which modulates the activity of the polypeptide.

23. An antibody substance which selectively binds to the polypeptide of claim 8, 9 or 10, wherein the antibody substance is made by providing the polypeptide to an immunocompetent vertebrate and thereafter harvesting blood or serum from the vertebrate.
